# EUROPEAN PATENT SPECIFICATION

(11) **EP 1 781 673 B1**
(45) Date of publication and mention of the grant of the patent: **21.10.2009**
(21) Application number: 05771781.1
(22) Date of filing: 16.08.2005
(51) Int. Cl.: C07D 513/04, C07D 498/04, A61K 31/519, A61P 35/00

(54) **SELECTED FUSED HETEROCYCLICS AND USES THEREOF**
AUSGEWÄHLTE KONDENSIERTE HETEROCYCLEN UND DEREN ANWENDUNG
COMPOSES HETEROCYCLIQUES EN FUSION SELECTIONNES ET UTILISATIONS DE CEUX-CI

(30) Priority: 18.08.2004 US 602399 P
(43) Date of publication of application: 09.05.2007
(73) Proprietor: AstraZeneca AB, 151 85 Södertälje (SE)
(72) Inventor: AQUILA, Brian AstraZeneca R & D Boston, Waltham, Massachusetts 02451 (US); BLOCK, Michael, Howard AstraZeneca R & D Boston, Waltham, Massachusetts 02451 (US); DAVIES, Audrey AstraZeneca R & D Boston, Waltham, Massachusetts 02451 (US); EZHUTHACHAN, Jayachandran AstraZeneca R & D Boston, Waltham, Massachusetts 02451 (US); PONTZ, Timothy AstraZeneca R & D Boston, Waltham, Massachusetts 02451 (US); RUSSELL, Daniel, John AstraZeneca R & D Boston, Waltham, Massachusetts 02451 (US); THEOCLITOU, Marie-Elena AstraZeneca R & D Boston, Waltham, Massachusetts 02451 (US); ZHENG, XiaoLan AstraZeneca R & D Boston, Waltham, Massachusetts 02451 (US)
(86) International application number: PCT/GB2005/003202
(87) International publication number: WO 2006/018627

(56) References cited:
- WO-A-03/049527
- WO-A-03/049679
- WO-A-20/04078758
- WO-A-20/04106492
- WO-A-20/04111058

## Description

### Field of the invention

The present invention relates to novel fused heterocycles, their pharmaceutical compositions and methods of use. In addition, the present invention relates to the use of these chemical compounds in the manufacture of a medicament for use in the treatment and prevention of cancers.

### Background of the invention

One sub-class of anti-cancer drugs (taxanes, vinca-alkaloids) now used extensively in the clinic is directed at microtubules and blocks the cell division cycle by interfering with normal assembly or disassembly of the mitotic spindle (see Chabner, B. A., Ryan, D. P., Paz-Ares, 1., Garcia-Carbonero, R., and Calabresi, P: Antineoplastic agents. In Hardman, J. G., Limbird, L.E., and Gilman, A., G., eds. Goodman and Gilman's The Pharmacological Basis of Therapeutics, 10th edition, 2001, The MacGraw-Hill Companies, Inc). Taxol® (paclitaxel), one of the most effective drugs of this class, is a microtubule stabilizer. It interferes with the normal growth and shrinkage of microtubules thus blocking cells in the metaphase of mitosis. Mitotic block is often followed by slippage into the next cell cycle without having properly divided, and eventually by apoptosis of these abnormal cells (Blagosklonny, M.V. and Fojo, T.: Molecular effects of paclitaxel: myths and reality (a critical review). Int J Cancer 1999, 83:151-156.).

Some of the side effects of treatment with paclitaxel are neutropenia and peripheral neuropathy. Paclitaxel is known to cause abnormal bundling of microtubules in interphase cells. In addition, some tumor types are refractory to treatment with paclitaxel, and other tumors become insensitive during treatment. Paclitaxel is also a substrate for the multi-drug resistance pump, P-glycoprotein ((see Chabner *et al.,* 2001).

Thus, there is a need for effective anti-mitotic agents that have fewer side effects than anti-microtubule drugs, and also for agents that are effective against taxane-resistant tumors. Kinesins are a large family of molecular motor proteins, which use the energy of adenosine 5'-triphosphate (ATP) hydrolysis to move in a stepwise manner along microtubules. For a review, see Sablin, E.P.: Kinesins and microtubules: their structures and motor mechanisms. Curr Opin Cell Biol 2000, 12:35-41 and Schief, W. R. and Howard, J.: Conformational changes during kinesin motility. Curr Opin Cell Biol 2001, 13:19-28.

Some members of this family transport molecular cargo along microtubules to the sites in the cell where they are needed. For example, some kinesins bind to vesicles and transport them along microtubules in axons. Several family members are mitotic kinesins, as they play roles in the reorganization of microtubules that establishes a bipolar mitotic spindle. The minus ends of the microtubules originate at the centrosomes, or spindle poles, whilst the plus ends bind to the kinetochore at the centromeric region of each chromosome. The mitotic spindle lines up the chromosomes at metaphase of mitosis and coordinates their movement apart and into individual daughter cells at anaphase and telophase (cytokinesis). See Alberts, B., Bray, D., Lewis, J., Raff, M., Roberts, K., and Watson, J. D., Molecular Biology of the Cell, 3rd edition, Chapter 18, The Mechanics of Cell Division, 1994, Garland Publishing, Inc. New York.

HsEg5 (homo sapiens Eg5) (Accession X85137; see Blangy, A., Lane H.A., d'Heron, P., Harper, M., Kress, M. and Nigg, E.A.: Phosphorylation by p34cdc2 regulates spindle association of human Eg5, a kinesin-related motor essential for bipolar spindle formation in vivo. Cell 1995, 83(7): 1159-1169) or, KSP (kinesin spindle protein), is a mitotic kinesin whose homologs in many organisms have been shown to be required for centrosome separation in the prophase of mitosis, and for the assembly of a bipolar mitotic spindle. For a review see Kashina, A.S., Rogers, G.C., and Scholey. J.M.: The bimC family of kinesins: essential bipolar mitotic motors driving centrosome separation. Biochem Biophys Acta 1997, 1357: 257-271. Eg5 forms a tetrameric motor, and it is thought to cross-link microtubules and participate in their bundling (Walczak, C. E., Vemos, I., Mitchison, T. J., Karsenti, E., and Heald, R.: A model for the proposed roles of different microtubule-based motor proteins in establishing spindle bipolarity. Curr Biol 1998, 8:903-913). Several reports have indicated that inhibition of Eg5 function leads to metaphase block in which cells display monastral spindles. Recently an Eg5 inhibitor called monastrol was isolated in a cell-based screen for mitotic blockers (Mayer, T.U., Kapoor, T. M., Haggarty, S.J., King, R.W., Schreiber, S.L., and Mitchison, T.J.: Small molecule inhibitor of mitotic spindle bipolarity identified in a phenotype-based screen. Science 1999, 286: 971-974).

Monastrol treatment was shown to be specific for Eg5 over kinesin heavy chain, another closely related motor with different functions (Mayer *et al.,* 1999). Monastrol blocks the release of ADP (adenosine 5'-diphosphate) from the Eg5 motor (Maliga, Z., Kapoor, T. M., and Mitchison, T.J.: Evidence that monastrol is an allosteric inhibitor of the mitotic kinesin Eg5. Chem & Biol 2002, 9: 989-996 and DeBonis, S., Simorre, J.-P., Crevel, I., Lebeau, L, Skoufias, D. A., Blangy, A., Ebel, C., Gans, P., Cross, R., Hackney, D. D., Wade, R. H., and Kozielski, F.: Interaction of the mitotic inhibitor monastrol with human kinesin Eg5. Biochemistry 2003, 42: 338-349) an important step in the catalytic cycle of kinesin motor proteins (for review, see Sablin, 2000; Schief and Howard, 2001). Treatment with monastrol was shown to be reversible and to activate the mitotic spindle checkpoint which stops the progress of the cell division cycle until all the DNA is in place for appropriate division to occur (Kapoor, T.M., Mayer, T. U., Coughlin, M. L., and Mitchison, T.J.: Probing spindle assembly mechanisms with monastrol, a small molecule inhibitor of the mitotic kinesin, Eg5. J Cell Biol 2000, 150(5): 975-988). Recent reports also indicate that inhibitors of Eg5 lead to apoptosis of treated cells and are effective against several tumor cell lines and tumor models (Mayer *et al.,* 1999).

Although Eg5 is thought to be necessary for mitosis in all cells, one report indicates that it is over-expressed in tumor cells (International Patent Application WO 01/31335), suggesting that they may be particularly sensitive to its inhibition. Eg5 is not present on the microtubules of interphase cells, and is targeted to microtubules by phosphorylation at an early point in mitosis (Blangy *et al.,* 1995), See also; Sawin, K. E, and Mitchison, T.J.: Mutations in the kinesin-like protein Eg5 disrupting localization to the mitotic spindle. Proc Natl Acad Sci USA 1995, 92(10): 4289-4293, thus monastrol has no detectable effect on microtubule arrays in interphase cells (Mayer et al., 1999). Another report suggests that Eg5 is involved in neuronal development in the mouse, but it disappears from neurons soon after birth, and thus Eg5 inhibition may not produce the peripheral neuropathy associated with treatment with paclitaxel and other anti-microtubule drugs (Ferhat, L., Expression of the mitotic motor protein Eg5 in postmitotic neurons: implications for neuronal development. J Neurosci 1998, 18(19): 7822-7835), Herein we describe the isolation of a class of specific and potent inhibitors of Eg5, expected to be useful in the treatment of neoplastic disease.

Certain pyrimidones have recently been described as being inhibitors of KSP (WO 03/094839, WO 03/099211, WO 03/050122, WHO 03/050064, WO 03/049679, WO 03/049527, WO 04/078758. WO 04/106492 and WHO 04/111058).

In accordance with the present invention, the present inventors have discovered novel chemical compounds which possess Eg5 inhibitory activity and are accordingly useful for their anti-cell-proliferation (such as anti-cancer) activity and are therefore useful in the manufacture of a medicament for use in the treatment of proliferative disorders, such as cancer.

### Summary of the invention

A compound selected from:
N-(3-amino-propyl)-N-{1-[5-(3-fluoro-benzyl)-3-methyl-4-oxo-4,5-dihydro-isothiazolo[5,4-d]pyrimidin-6-yl]-propyl}-4-methyl-benzamide;
N-(3-amino-propyl)-N-{1-[5-(3-fluoro-benzyl)-3-methyl-4-oxo-4,5-dihydro-isothiazolo[5,4-d]pyrimidin-6-yl]-2-methyl-propyl}-4-methyl-benzamide;
N-(2,amino-ethyl)-N-[1-(5-benzyl-3-methyl-4-oxo-4,5-dihydro-isothiazolo[5,4-d]pyrimidin-6-yl)-2-methyl-propyl]-4-bromo-benzamide;
N-(2-amino-ethyl)-N-[1-(5-benzyl-3-methyl-4-oxo-4,5-dihydro-isothiazolo[5,4-d]pyrimidin-6-yl)-2-methyl-propyl]-4-methyl-benzamide;
N-(2-amino-ethyl}-N-[1-(5-benzyl-3-methyl-4-oxo-4,5-dihydro-isothiazolo[5,4-d]pyrimidin-6-yl)-2-methyl-propyl]-3-fluoro-4-methyl-benzamide;
N-(3-amino-propyl)-N-[1-(5-benzyl-3-methyl-4-oxo-4,5-dihydro-isothiazolo[5,4-d]pyrimidin-6-yl)-2-methyl -propyl]-3-fluoro-4-methyl-benzamide;
N-(3-amino-propyl)-N-[1-(5-benzyl-3-methyl-4-oxo-4,5-dihydro-isothiazolo[5,4-d]pyrimidin-6-yl)-2-methyl-propyl]-4-bromo-benzamide;
N-[1-(5-benzyl-3-methyl-4-oxo-4,5-dihydro-isothiazolo[5,4-d]pyrimidin-6-yl)-2-methyl-propyl]-N-(3-dimethylamino-propyl)-4-methyl-benzamide;
N-[1-(5-benzyl-3-methyl-4-oxo-4,5-dihydro-isothiazolo[5,4-d]pyrimidin-6-yl)-2-methyl-propyl]-N-(3-dimethylamino-propyl)-4-bromo-benzamide;
N-[1-(5-benzyl-3-methyl-4-oxo-4,5-dihydro-isothiazolo[5,4-d]pyimidin-6-yl)-2-methyl-propyl]-N-(3-dimethylamino-propyl)-3-fluoro-4-methyl-benzamide;
N-(3-amino-propyl)-N-{1-[5-(3-fluoro-benzyl)-3-methyl-4-oxo-4,5-dihydro-isoxazolo [5,4-d]pyrimidin-6-yl]-2-methyl-propyl}-4-methyl-benzamide;
N-(3-amino-propyl)-N-[1-(6-benzyl-3-methyl-7-oxo-6,7-dihydro-isothiazolo[4,5-d]pyrimidin-5-yl)-propyl]-4-methyl-benzamide.

The invention also encompasses stereoisomers, enantiomers, in vivo-hydrolysable precursors and pharmaceutically-acceptable salts of compounds of formula (I), pharmaceutical compositions and formulations containing them, methods of using them to treat diseases and conditions either alone or in combination with other therapeutically-active compounds or substances, processes and intermediates used to prepare them, uses of them as medicaments, uses of them in the manufacture of medicaments and uses of them for diagnostic and analytic purposes.

### Detailed description of the invention

In a first embodiment, the present invention provides a novel compound selected from:
N-(3-amino-propyl)-N-{1-[5-(3-fluoro-benzyl)-3-methyl-4-oxo-4,5-dihydro-isothiazolo[5,4-d]pyrimidin-6-yl]-propyl}-4-methyl-benzamide;
N-(3-amino-propyl)-N-{1-[5-(3-fluoro-benzyl)-3-methyl-4-oxo-4,5-dihydro-isothiazolo[5,4-d]pyrimidin-6-yl]-2-methyl-propyl}-4-methyl-benzamide;
N-(2-amino-ethyl)-N-[1-(5-benzyl-3-methyl-4-oxo-4,5-dihydro-isothiazolo[5,4-d]pyrimidin-6-yl)-2-methyl-propyl]-4-bromo-benzamide;
N-(2-amino-ethyl)-N-[1-(5-benzyl-3-methyl-4-oxo-4,5-dihydro-isothiazolo[5,4-d]pyrimidin-6-yl)-2-methyl-propyl]-4-methyl-benzamide;
N-(2-amino-ethyl)-N-[1-(5-benzyl-3-methyl-4-oxo-4,5-dihydro-isothiazolo[5,4-d]pyrimidin-6-yl)-2-methyl-propyl]-3-fluoro-4-methyl-benzamide;
N-(3-amino-propyl)-N-[1-(5-benzyl-3-methyl-4-oxo-4,5-dihydro-isothiazolo[5,4-d]pyrimidin-6-yl)-2-methyl-propyl]-3-fluoro-4-methyl-benzamide;
N-(3-amino-propyl)-N-[1-(5-benzyl-3-methyl-4-oxo-4,5-dihydro-isothiazolo[5,4-d]pyrimidin-6-yl)-2-methyl-propyl]-4-bromo-benzamide;
N-[1-(5-benzyl-3-methyl-4-oxo-4,5-dihydro-isothiazolo[5,4-d]pyrimidin-6-yl)-2-methyl-propyl]-N-(3-dimethylamino-propyl)-4-methyl-benzamide;
N-[1-(5-benzyl-3-methyl-4-oxo-4,5-dihydro-isothiazolo[5,4-d]pyrimidin-6-yl)-2-methyl-propyl]-N-(3-dimethylamino-propyl)-4-bromo-benzamide;
N-[1-(5-benzyl-3-methyl-4-oxo-4,5-dihydro-isothiazolo[5,4-d]pyrimidin-6-yl)-2-methyl-propyl]-N-(3-dimethylamino-propyl)-3-fluoro-4-methyl-benzamide;
N-(3-amino-propyl)-N-{1-[5-(3-fluoro-benzyl)-3-methyl-4-oxo-4,5-dihydro-isoxazolo [5,4-d]pyrimidin-6-yl]-2-methyl-propyl}-4-methyl-benzamide;
N-(3-amino-propyl)-N-[1-(6-benzyl-3-methyl-7-oxo-6,7-dihydro-isothiazolo[4,5-d]pyrimidin-5-yl)-propyl]-4-methyl-benzamide,

In a further aspect of the invention there is provided a compound selected from the following:
N-(3-Amino-propyl)-N-{1-[5-(3-fluoro-benzyl)-3-methyl-4-oxo-4,5-dihydro-isothiazolo[5,4-d]pyrimidin-6-yl]-propyl}-4-methyl-benzamide hydrogen chloride;
N-(3-Amino-propyl)-N-{1-[5-(3-fluoro-benzyl)-3-methyl-4-oxo-4,5-dihydro-isothiazolo[5,4-d]pyrimidin-6-yl}-2-methyl-propyl}-4-methyl-benzamide hydrogen chloride;
N-(2-Amino-ethyl)-N-[1-(5-benzyl-3-methyl-4-oxo-4,5-dihydro-isothiazolo[5,4-d]pyrimidin-6-yl)-2-methyl-propyl]-4-bromo-benzamide hydrogen chloride;
N-(2-Amino-ethyl)-N-[1-(5-benzyl-3-methyl-4-oxo-4,5-dihydro-isothiazolo[5,4-d]pyrimidin-6-yl)-2-methyl-propyl]-4-methyl-benzamide hydrogen chloride;
N-(2-Amino-ethyl)-N-[1-(5-benzyl-3-methyl-4-oxo-4,5-dihydro-isothiazolo[5,4-d]pyrimidin-6-yl)-2-methyl-propyl]-3-fluoro-4-methyl-benzamide hydrogen chloride;
N-(3-Amino-propyl)-N-[1-(5-benzyl-3-methyl-4-oxo-4,5-dihydro-isothiazolo[5,4-d]pyrimidin-6-yl)-2-methyl-propyl]-3-fluoro-4-methyl-benzamide hydrogen chloride;
N-(3-Amino-propyl)-N-[1-(5-benzyl-3-methyl-4-oxo-4,5-dihydro-isothiazolo[5,4-d]pyrimidin-6-yl)-2-methyl-propyl]-4-bromo-benzamide hydrogen chloride;
N-[1-(5-Benzyl-3-methyl-4-oxo-4,5-dihydro-isothiazolo[5,4-d]pyrimidin-6-yl)-2-methyl-propyl]-N-(3-dimethylamino-propyl)-4-methyl-benzamide hydrogen chloride;
N-[1-(5-Benzyl-3-methyl-4-oxo-4,5-dihydro-isothiazolo[5,4-d]pyrimidin-6-yl)-2-methyl-propyl]-N-(3-dimethylamino-propyl)-4-bromo-benzamide hydrogen chloride;
N-[1-(5-Benzyl-3-methyl-4-oxo-4,5-dihydro-isothiazolo[5,4-d]pyrimidin-6-yl)-2-methyl-propyl]-N-(3-dimethylamino-propyl)-3-fluoro-4-methyl-benzamide hydrogen chloride;
N-(3-Amino-propyl)-N-{1-[5-(3-fluoro-benzyl)-3-methyl-4-oxo-4,5-dihydro-isoxazolo [5,4-d]pyrimidin-6-yl}-2-methyl-propyl}-4-methyl-benzamide hydrogen chloride;
N-(3-Amino-propyl)-N-[1-(6-benzyl-3-methyl-7-oxo-6,7-dihydro-isothiazolo[4,5-d]pyrimidin-5-yl)-propyl]-4-methyl-benzamide hydrogen chloride,
or a pharmaceutically acceptable salt thereof.

In a further embodiment the present invention provides a compound of formula (I) or a pharmaceutically acceptable salt or an *in vivo* hydrolysable ester thereof for use as a medicament.

In a further embodiment the present invention provides a compound of formula (I) or a pharmaceutically acceptable salt thereof for use as a medicament.

According to a further aspect of the invention there is provided the use of a compound of the formula **(I),** or a pharmaceutically acceptable salt thereof, as defined hereinbefore in the manufacture of a medicament for use in the production of an Eg5 inhibitory effect in a warm-blooded animal such as man.

According to a further aspect of the invention there is provided the use of a compound of the formula **(I),** or a pharmaceutically acceptable salt thereof, as defined hereinbefore in the manufacture of a medicament for use in the production of an anti-proliferative effect in a warm-blooded animal such as man.

According to this aspect of the invention there is provided the use of a compound of the formula **(I),** or a pharmaceutically acceptable salt thereof, as defined hereinbefore in the manufacture of a medicament for use in the production of an anti-cancer effect in a warm-blooded animal such as man.

According to a further feature of the invention, there is provided the use of a compound of the formula **(I),** or a pharmaceutically acceptable salt thereof, as defined herein before in the manufacture of a medicament for use in the treatment of carcinomas of the brain, breast, ovary, lung, colon and prostate, multiple myeloma, leukemias, lymphomas, tumors of the central and peripheral nervous system, melanoma, fibrosarcoma, Ewing's sarcoma and osteosarcoma.

In a further embodiment the present invention provides a compound of formula **(I)** or a pharmaceutically acceptable salt or an *in vivo* hydrolysable ester thereof, in the manufacture of a medicament for the treatment or prophylaxis of disorders associated with cancer.

In a further embodiment the present invention provides a compound of formula **(I)** or a pharmaceutically acceptable salt thereof, in the manufacture of a medicament for the treatment or prophylaxis of disorders associated with cancer.

In a further embodiment the present invention provides a pharmaceutical composition comprising a compound of formula **(I)** or a pharmaceutically acceptable salt or an *in vivo* hydrolysable ester thereof together with at least one pharmaceutically acceptable carrier, diluent or excipient.

In a further embodiment the present invention provides a pharmaceutical composition comprising a compound of formula **(I)** or a pharmaceutically acceptable salt thereof together with at least one pharmaceutically acceptable carrier, diluent or excipient.

In a further aspect of the invention there is provided a pharmaceutical composition which comprises a compound of the formula **(I),** or a pharmaceutically acceptable salt thereof, as defined herein before in association with a pharmaceutically-acceptable diluent or carrier for use in the production of an Eg5 inhibitory effect in a warm-blooded animal such as man.

In a further aspect of the invention there is provided a pharmaceutical composition which comprises a compound of the formula **(I),** or a pharmaceutically acceptable salt thereof, as defined herein before in association with a pharmaceutically-acceptable diluent or carrier for use in the production of an anti-proliferative effect in a warm-blooded animal such as man.

In a further aspect of the invention there is provided a pharmaceutical composition which comprises a compound of the formula **(I),** or a pharmaceutically acceptable salt thereof, as defined herein before in association with a pharmaceutically-acceptable diluent or carrier for use in the production of an anti-cancer effect in a warm-blooded animal such as man.

In a further aspect of the invention there is provided a pharmaceutical composition which comprises a compound of the formula **(I),** or a pharmaceutically acceptable salt thereof, as defined herein before in association with a pharmaceutically-acceptable diluent or carrier for use in the treatment of carcinomas of the brain, breast, ovary, lung, colon and prostate, multiple myeloma, leukemias, lymphomas, tumors of the central and peripheral nervous system, melanoma, fibrosarcoma, Ewing's sarcoma and osteosarcoma in a warm-blooded animal such as man.

According to a further aspect of the invention there is provided the use of a compound of the formula (I), or a pharmaceutically acceptable salt thereof, as defined hereinbefore in the production of an Eg5 inhibitory effect in a warm-blooded animal such as man.

According to a further aspect of the invention there is provided the use of a compound of the formula (I), or a pharmaceutically acceptable salt thereof, as defined hereinbefore for use in the production of an anti-proliferative effect in a warm-blooded animal such as man.

According to this aspect of the invention there is provided the use of a compound of the formula (I), or a pharmaceutically acceptable salt thereof, as defined hereinbefore for use in the production of an anti-cancer effect in a warm-blooded animal such as man.

According to a further feature of the invention, there is provided the use of a compound of the formula (I), or a pharmaceutically acceptable salt thereof, as defined herein before for use in the treatment of carcinomas of the brain, breast, ovary, lung, colon and prostate, multiple myeloma, leukemias, lymphomas, tumors of the central and peripheral nervous system, melanoma, fibrosarcoma, Ewing's sarcoma and osteosarcoma.

According to a further aspect of the invention there is provided the use of a compound of the formula (I), or a pharmaceutically acceptable salt thereof, as defined hereinbefore in the production of an Eg5 inhibitory effect in a warm-blooded animal such as man.

According to a further aspect of the invention there is provided the use of a compound of the formula (I), or a pharmaceutically acceptable salt thereof, as defined hereinbefore for use in the production of an anti-proliferative effect in a warm-blooded animal such as man.

According to this aspect of the invention there is provided the use of a compound of the formula (I), or a pharmaceutically acceptable salt thereof, as defined hereinbefore for use in the production of an anti-cancer effect in a warm-blooded animal such as man.

According to a further feature of the invention, there is provided the use of a compound of the formula (I), or a pharmaceutically acceptable salt thereof, as defined herein before for use in the treatment of carcinomas of the brain, breast, ovary, lung, colon and prostate, multiple myeloma, leukemias, lymphomas, tumors of the central and peripheral nervous system, melanoma, fibrosarcoma, Ewing's sarcoma and osteosarcoma.

The definitions set forth in this section are intended to clarify terms used throughout this application. The term "herein" means the entire application.

"RT" or "rt" means room temperature.

When any variable (e.g., R¹, R⁴ etc.) occurs more than one time in any constituent or formula for a compound, its definition at each occurrence is independent of its definition at every other occurrence. Thus, for example, if a group is shown to be substituted with 0-3 R¹, then said group may optionally be substituted with 0,1, 2 or 3 R¹ groups and R¹ at each occurrence is selected independently from the definition of R¹. Also, combinations of substituents and/or variables are permissible only if such combinations result in stable compounds.

A variety of compounds in the present invention may exist in particular geometric or stereoisomeric forms. The present invention takes into account all such compounds, including cis- and trans isomers, R- and S- enantiomers, diastereomers, (D)-isomers, (L)-isomers, the racemic mixtures thereof, and other mixtures thereof, as being covered within the scope of this invention. Additional asymmetric carbon atoms may be present in a substituent such as an alkyl group. All such isomers, as well as mixtures thereof, are intended to be included in this invention. The compounds herein described may have asymmetric centers. Compounds of the present invention containing an asymmetrically substituted atom may be isolated in optically active or racemic forms. It is well known in the art how to prepare optically active forms, such as by resolution of racemic forms or by synthesis from optically active starting materials. When required, separation of the racemic material can be achieved by methods known in the art. Many geometric isomers of olefins, C=N double bonds, and the like can also be present in the compounds described herein, and all such stable isomers are contemplated in the present invention. Cis and trans geometric isomers of the compounds of the present invention are described and may be isolated as a mixture of isomers or as separated isomeric forms. All chiral, diastereomeric, racemic forms and all geometric isomeric forms of a structure are intended, unless the specific stereochemistry or isomeric form is specifically indicated.

When a bond to a substituent is shown to cross a bond connecting two atoms in a ring, then such substituent may be bonded to any atom on the ring. When a substituent is listed without indicating the atom via which such substituent is bonded to the rest of the compound of a given formula, then such substituent may be bonded via any atom in such substituent. Combinations of substituents and/or variables are permissible only if such combinations result in stable compounds.

As used herein, "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

As used herein, "pharmaceutically acceptable salts" refer to derivatives of the disclosed compounds wherein the parent compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like. The pharmaceutically acceptable salts include the conventional non-toxic salts or the quaternary animonium salts of the parent compound formed, for example, from non-toxic inorganic or organic acids. For example, such conventional non-toxic salts include those derived from inorganic acids such as hydrochloric, phosphoric, and the like; and the salts prepared from organic acids such as lactic, maleic, citric, benzoic, methanesulfonic, and the like. The pharmaceutically acceptable salts of the invention also include salts prepared with one of the following acids benzene sulfonic acid, fumaric acid, methanesulfonic acid, naphthalene-1,5-disulfonic acid, naphthalene-2-sulfonic acid or L-tartaric acid.

Thus in one aspect of the invention there is provided a compound of the invention, particularly one of the Examples described herein, as a pharmaceutically acceptable salt, particularly a benzene sulfonic acid, fumaric acid, methanesulfonic acid, naphthalene-1,5-disulfonic acid, naphthalene-2-sulfonic acid or L-tartaric acid salt.

The pharmaceutically acceptable salts of the present invention can be synthesized from the parent compound that contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, nonaqueous media like ether, ethyl acetate, ethanol, isopropanol, or acetonitrile are preferred.

As used herein, "in vivo hydrolysable ester" means an in vivo hydrolysable (or cleavable) ester of a compound of the formula (I) that contains a carboxy or a hydroxy group. For example amino acid esters, C₁₋₆alkoxymethyl esters like methoxymethyl; C₁₋₆alkanoyloxymethyl esters like pivaloyloxymethyl; C₃₋₈cycloalkoxycarbonyloxy C₁₋₆alkyl esters like 1-cyclohexylcarbonyloxyethyl, acetoxymethoxy, or phosphoramidic cyclic esters.

All chemical names were generated using a software system known as AutoNom Name accessed through ISIS draw.

### Combinations

The anti-cancer treatment defined herein may be applied as a sole therapy or may involve, in addition to the compound of the invention, conventional surgery or radiotherapy or chemotherapy. Such chemotherapy may include one or more of the following categories of antitumour agents:
(i) antiproliferative/antineoplastic drugs and combinations thereof, as used in medical oncology, such as alkylating agents (for example cis-platin, carboplatin, oxaliplatin, cyclophosphamide, nitrogen mustard, melphalan, chlorambucil, busulphan, temozolomide and nitrosoureas); antimetabolites (for example gemcitabine and antifolates such as fluoropyrimidines like 5-fluorouracil and tegafur, raltitrexed, methotrexate, cytosine arabinoside and hydroxyurea); antitumour antibiotics (for example anthracyclines like adriamycin, bleomycin, doxorubicin, daunomycin, epirubicin, idarubicin, mitomycin-C, dactinomycin and mithramycin); antinitotic agents (for example vinca alkaloids like vincristine, vinblastine, vindesine and vinorelbine and taxoids like taxol and taxotere) polokinase inhibitors; and topoisomerase inhibitors (for example epipodophyllotoxins like etoposide and teniposide, amsacrine, topotecan and camptothecin);
(ii) cytostatic agents such as antioestrogens (for example tamoxifen, toremifene, raloxifene, droloxifene and iodoxyfene), oestrogen receptor down regulators (for example fulvestrant), antiandrogens (for example bicalutamide, flutamide, nilutamide and cyproterone acetate), LHRH antagonists or LHRH agonists (for example goserelin, leuprorelin and buserelin), progestogens (for example megestrol acetate), aromatase inhibitors (for example as anastrozole, letrozole, vorazole and exemestane) and inhibitors of 5α-reductase such as finasteride;
(iii) agents which inhibit cancer cell invasion (for example metalloproteinase inhibitors like marimastat and inhibitors of urokinase plasminogen activator receptor function or inhibitors of SRC kinase (like 4-(6-chloro-2,3-methylenedioxyanilino)-7-[2-(4-methylpiperazin-1-yl)ethoxy]-5-tetrahydropyran-4-yloxyqyuinazoline (AZD0530; International Patent Application WO 01/94341) and N-(2-chloro-6-methylphenyl)-2-{6-[4-(2-hydroxyethyl)piperazin-1-yl]-2-methylpyrimidin-4-ylamino}thiazole-5-carboxamide (dasatinib, BMS-354825; J. Med. Chem., 2004, 47, 6658-6661))or antibodies to Heparanase);
(iv) inhibitors of growth factor function, for example such inhibitors include growth factor antibodies, growth factor receptor antibodies (for example the anti-erbb2 antibody trastuzumab [Herceptin™] and the anti-erbb1 antibody cetuximab [Erbitux, C225]), Ras/Raf signalling inhibitors such as farnesyl transferase inlubitors(for example sorafenib (BAY 43-9006) and tipifarnib), tyrosine kinase inhibitors and serine/threonine kinase inhibitors, for example inhibitors of the epidermal growth factor family (for example EGFR family tyrosine kinase inhibitors such as N-(3-chloro-4-fluorophenyl)-7-methoxy-6-(3-morpholinopropoxy)quinazolin-4-amine (gefitinib, AZD1839), N-(3-ethynylphenyl)-6,7-bis(2-methoxyethoxy)quinazolin-4-amine (erlotinib, OSI-774) and 6-acrylamido-N-(3-chloro-4-fluorophenyl)-7-(3-morpholinopropoxy)quinazolin-4-amine (CI 1033) and erbB2 tyrosine kinase inhibitors such as lapatinib), for example inhibitors of the platelet-derived growth factor family such as imatinib, and for example inhibitors of the hepatocyte growth factor family, c-kit inhibitors, ab1 kinase inhibitors, IGF receptor (insulin-like growth factor) kinase inhibitors and inhibitors of cell signalling through MEK, AKT and/or PI3K kinases;
(v) antiangiogenic agents such as those which inhibit the effects of vascular endothelial growth factor, (for example the anti-vascular endothelial cell growth factor antibody bevacizumab [Avastin^{™}], and VEGF receptor tyrosine kinase inhibitors such as those disclosed in International Patent Applications WO 97/22596, WO 97/30035, WO 97/32556, WO 98/13354, 4-(4-bromo-2-fluoroanilino)-6-methoxy-7-(1-methylpiperidin-4-ylmethoxy)quinazoline (ZD6474; Example 2 within WO 01/32651), 4-(4-fluoro-2-methylindol-5-yloxy)-6-methoxy-7-(3-pyrrolidin-1-ylpropoxy)quinazoline (AZD2171; Example 240 within WO 00/47212), vatalanib (PTK787; WO 98/35985) and SU11248 (sunitinib; WO 01/60514)) and compounds that work by other mechanisms (for example linomide, inhibitors of integrin αvβ3 function and angiostatin), ang1 and 2 inhibitors;
(vi) vascular damaging agents such as Combretastatin A4 and compounds disclosed in International Patent Applications WO 99/02166, two 00/40529, WO 00/41669, WO 01/92224, WO 02/04434 and WO 02/08213, anti bcl2;
(vii) antisense therapies, for example those which are directed to the targets listed above, such as ISIS 2503, an anti-ras antisense;
(viii) gene therapy approaches, including for example approaches to replace aberrant genes such as aberrant p53 or aberrant BRCA1 or BRCA2, GDEPT (gene-directed enzyme pro-drug therapy) approaches such as those using cytosine deaminase, thymidine kinase or a bacterial nitroreductase enzyme and approaches to increase patient tolerance to chemotherapy or radiotherapy such as multi-drug resistance gene therapy;
(ix) immunotherapy approaches, including for example ex-vivo and in-vivo approaches to increase the immunogenicity of patient tumour cells, such as transfection with cytokines such as interleukin 2, interleukin 4 or granulocyte-macrophage colony stimulating factor, approaches to decrease T-cell anergy, approaches using transfected immune cells such as cytokine-transfected dendritic cells, approaches using cytokine-transfected tumour cell lines and approaches using anti-idiotypic antibodies; x) cell cycle agents such as aurora kinase inhibitors (for example PH739358, VX-680, MLN8054, R763, MP235, MP529, VX-528, AX39459 and the specific examples mentioned in WO02/00649 WO03/055491, WO2004/058752, WO2004/058781, WO2004/058782, WO2004/094410 WO2004/105764, WO2004/113324 which are incorporated herein by reference), and cyclin dependent kinase inhibitors such as CDK2 and/or CDK4 inhibitors (for example the specific examples of WO01/14375, WO01/72717, WO02/04429, WO02/20512, WO02/66481, WO02/096887, WO03/076435, WO03/076436, WO03/076434, WO03/076433, WO04/101549 and WO04/101564 which are incorporated herein by reference); and xi) cytotoxic agents such as gemcitibine, topoisomerase 1 inhibitors (adriamycin, etoposide) and topoisomerase II inhibitors.

Such conjoint treatment may be achieved by way of the simultaneous, sequential or separate dosing of the individual components of the treatment. Such combination products employ the compounds of this invention within the dosage range described hereinbefore and the other pharmaceutically-active agent within its approved dosage range.

In a further aspect of the present invention there is provided a compound of formula (I) or a pharmaceutically acceptable salt or *an in vivo* hydrolysable ester thereof in combination with simultaneous, sequential or separate dosing of an anti-tumor agent or class selected from the list herein above.

Therefore in a further embodiment the present invention provides a method for the treatment of cancer by administering to a human a compound of formula (I) or a pharmaceutically acceptable salt or an *in vivo* hydrolysable ester thereof in combination with simultaneous, sequential or separate dosing of an anti-tumor agent or class selected from the list herein above.

In a further aspect of the present invention there is provided the use of a compound of formula (I) or a phannaceutically acceptable salt or an *in vivo* hydrolysable ester thereof in combination with simultaneous, sequential or separate dosing of an anti-tumor agent or class selected from the list herein above for use in the manufacture of a medicament for use in the treatment of cancer.

In a further aspect of the present invention there is provided the use of a compound of formula (I) or a pharmaceutically acceptable salt or an *in vivo* hydrolysable ester thereof in combination with simultaneous, sequential or separate dosing of an anti-tumor agent or class selected from the list herein above for use in the treatment of cancer.

The anti-cancer treatment defined herein may also include one or more of the following categories of pharmaceutical agents:
i) an agent useful in the treatment of anemia, for example, a continuous eythropoiesis receptor activator (such as epoetin alfa);
ii) an agent useful in the treatment of neutropenia, for example, a hematopoietic growth factor which regulates the production and function of neutroplils such as a human granulocyte colony stimulating factor, (G-CSF), for example filgrastim; and
iii) an anti-emetic agent to treat nausea or emesis, including acute, delayed, late-phase, and anticipatory emesis, which may result from the use of a compound of the present invention, alone or with radiation therapy, suitable examples of such anti emetic agents include neurokinin-1 receptor antagonists, 5H13 receptor antagonists, such as ondansetron, granisetron, tropisetron, and zatisetron, GABAB receptor agonists, such as baclofen, a corticosteroid such as Decadron (dexamethasone), Kenalog, Aristocort, Nasalide, Preferid or Benecorten, an antidopaminergic, such as the phenothiazines (for example prochlorperazine, fluphenazine, thioridazine and mesoridazine), metoclopramide or dronabinol.

Such conjoint treatment may be achieved by way of the simultaneous, sequential or separate dosing of the individual components of the treatment. Such conjoint treatment employs the compounds of this invention within the dosage range described hereinbefore and the other pharmaceutically-active agent within its approved dosage range.

In a further aspect of the present invention there is provided a compound of formula (I) or a pharmaceutically acceptable salt or an *in vivo* hydrolysable ester thereof in combination with simultaneous, sequential or separate dosing of another pharmaceutical agent or class selected from the list herein above.

In a further aspect of the present invention there is provided the use of a compound of formula (I) or a pharmaceutically acceptable salt or an *in vivo* hydrolysable ester thereof in combination with simultaneous, sequential or separate dosing of another pharmaceutical agent or class selected from the list herein above for use in the manufacture of a medicament for use in the treatment of cancer.

In a further aspect of the present invention there is provided the use of a compound of formula (I) or a pharmaceutically acceptable salt or an *in vivo* hydrolysable ester thereof in combination with simultaneous, sequential or separate dosing of another pharmaceutical agent or class selected from the list herein above for use in the treatment of cancer.

In addition to their use in therapeutic medicine, the compounds of formula (I) and their pharmaceutically acceptable salts are also useful as pharmacological tools in the development and standardisation of in vitro and *in vivo* test systems for the evaluation of the effects of inhibitors of Eg5 in laboratory animals such as cats, dogs, rabbits, monkeys, rats and mice, as part of the search for new therapeutic agents.

In the above other pharmaceutical composition, process, method, use and medicament manufacture features, the alternative and preferred embodiments of the compounds of the invention described herein also apply.

### Formulations

Compounds of the present invention may be administered orally, parenteral, buccal, vaginal, rectal, inhalation, insufflation, sublingually, intramuscularly, subcutaneously, topically, intranasally, intraperitoneally, intrathoracially, intravenously, epidurally, intrathecally, intracerebroventricularly and by injection into the joints.

The dosage will depend on the route of administration, the severity of the disease, age and weight of the patient and other factors normally considered by the attending physician, when determining the individual regimen and dosage level as the most appropriate for a particular patient.

An effective amount of a compound of the present invention for use in therapy of infection is an amount sufficient to symptomatically relieve in a warm-blooded animal, particularly a human the symptoms of infection, to slow the progression of infection, or to reduce in patients with symptoms of infection the risk of getting worse.

For preparing pharmaceutical compositions from the compounds of this invention, inert, pharmaceutically acceptable carriers can be either solid or liquid. Solid form preparations include powders, tablets, dispersible granules, capsules, cachets, and suppositories.

A solid carrier can be one or more substances, which may also act as diluents, flavoring agents, solubilizers, lubricants, suspending agents, binders, or tablet disintegrating agents; it can also be an encapsulating material.

In powders, the carrier is a finely divided solid, which is in a mixture with the finely divided active component. In tablets, the active component is mixed with the carrier having the necessary binding properties in suitable proportions and compacted in the shape and size desired.

For preparing suppository compositions, a low-melting wax such as a mixture of fatty acid glycerides and cocoa butter is first melted and the active ingredient is dispersed therein by, for example, stirring. The molten homogeneous mixture is then poured into convenient sized molds and allowed to cool and solidify.

Suitable carriers include magnesium carbonate, magnesium stearate, talc, lactose, sugar, pectin, dextrin, starch, tragacanth, methyl cellulose, sodium carboxymethyl cellulose, a low-melting wax, cocoa butter, and the like.

Some of the compounds of the present invention are capable of forming salts with various inorganic and organic acids and bases and such salts are also within the scope of this invention. Examples of such acid addition salts include acetate, adipate, ascorbate, benzoate, benzenesulfonate, bicarbonate, bisulfate, butyrate, camphorate, camphorsulfonate, choline, citrate, cyclohexyl sulfamate, diethylenediamine, ethanesulfonate, fumarate, glutamate, glycolate, hemisulfate, 2-hydroxyethylsulfonate, heptanoate, hexanoate, hydrochloride, hydrobronide, hydroiodide, hydroxymaleate, lactate, malate, maleate, methanesulfonate, meglumine, 2-naphthalenesulfonate, nitrate, oxalate, pamoate, persulfate, phenylacetate, phosphate, diphosphate, picrate, pivalate, propionate, quinate, salicylate, stearate, succinate, sulfamate, sulfanilate, sulfate, tartrate, tosylate (p-toluenesulfonate), trifluoroacetate, and undecanoate. Base salts include ammonium salts, alkali metal salts such as sodium, lithium and potassium salts, alkaline earth metal salts such as aluminum, calcium and magnesium salts, salts with organic bases such as dicyclohexylamine salts, N-methyl-D-glucamine, and salts with amino acids such as arginine, lysine, ornithine, and so forth. Also, basic nitrogen-containing groups may be quaternized with such agents as: lower alkyl halides, such as methyl, ethyl, propyl, arid butyl halides; dialkyl sulfates like dimethyl, diethyl, dibutyl; diamyl sulfates; long chain halides such as decyl, lauryl, myristyl and stearyl halides; aralkyl halides like benzyl bromide and others. Non-toxic physiologically-acceptable salts are preferred, although other salts are also useful, such as in isolating or purifying the product.

The salts may be formed by conventional means, such as by reacting the free base form of the product with one or more equivalents of the appropriate acid in a solvent or medium in which the salt is insoluble, or in a solvent such as water, which is removed *in vacuo* or by freeze drying or by exchanging the anions of an existing salt for another anion on a suitable ion-exchange resin.

In order to use a compound of the formula **(**I) or a pharmaceutically acceptable salt thereof for the therapeutic treatment (including prophylactic treatment) of mammals including humans, it is normally formulated in accordance with standard pharmaceutical practice as a pharmaceutical composition.

In addition to the compounds of the present invention, the pharmaceutical composition of this invention may also contain, or be co-administered (simultaneously or sequentially) with, one or more pharmacological agents of value in treating one or more disease conditions referred to herein.

The term composition is intended to include the formulation of the active component or a pharmaceutically acceptable salt with a pharmaceutically acceptable carrier. For example this invention may be formulated by means known in the art into the form of, for example, tablets, capsules, aqueous or oily solutions, suspensions, emulsions, creams, ointments, gels, nasal sprays, suppositories, finely divided powders or aerosols or nebulisers for inhalation, and for parenteral use (including intravenous, intramuscular or infusion) sterile aqueous or oily solutions or suspensions or sterile emulsions.

Liquid form compositions include solutions, suspensions, and emulsions. Sterile water or water-propylene glycol solutions of the active compounds may be mentioned as an example of liquid preparations suitable for parenteral administration. Liquid compositions can also be formulated in solutions in aqueous polyethylene glycol solution. Aqueous solutions for oral administration can be prepared by dissolving the active component in water and adding suitable colorants, flavoring agents, stabilizers, and thickening agents as desired. Aqueous suspensions for oral use can be made by dispersing the finely divided active component in water together with a viscous material such as natural synthetic gums, resins, methyl cellulose, sodium carboxymethyl cellulose, and other suspending agents known to the pharmaceutical formulation art.

The pharmaceutical, compositions can be in unit dosage form. In such form, the composition is divided into unit doses containing appropriate quantities of the active component. The unit dosage form can be a packaged preparation, the package containing discrete quantities of the preparations, for example, packeted tablets, capsules, and powders in vials or ampoules. The unit dosage form can also be a capsule, cachet, or tablet itself, or it can be the appropriate number of any of these packaged forms.

### Synthesis

The compounds of the present invention can be prepared in a number of ways well known to one skilled in the art of organic synthesis. The compounds of the present invention can be synthesized using the methods described herein, together with synthetic methods known in the art of synthetic organic chemistry, or variations thereon as appreciated by those skilled in the art. Such methods include, but are not limited to, those described herein.

The novel compounds of this invention may be prepared using the reactions and techniques described herein. The reactions are performed in solvents appropriate to the reagents and materials employed and are suitable for the transformations being effected. Also, in the description of the synthetic methods described herein, it is to be understood that all proposed reaction conditions, including choice of solvent, reaction atmosphere, reaction temperature, duration of the experiment and workup procedures, are chosen to be the conditions standard for that reaction, which should be readily recognized by one skilled in the art. It is understood by one skilled in the art of organic synthesis that the functionality present on various portions of the molecule must be compatible with the reagents and reactions proposed. Such restrictions to the substituents, which are compatible with the reaction conditions, will be readily apparent to one skilled in the art and alternate methods must then be used.

The starting materials for the examples contained herein are either commercially available or are readily prepared by standard methods from known materials. For example the following reactions are illustrations of the preparation of some of the starting materials and examples used herein.

### Examples

The invention will now be illustrated by the following examples in which, unless stated otherwise:
(i) temperatures are given in degrees Celsius (°C); operations were carried out at room or ambient temperature, that is, at a temperature in the range of 18-30°C;
(ii) organic solutions were dried over anhydrous sodium sulphate; evaporation of solvent was carried out using a rotary evaporator under reduced pressure (600-4000 Pascals; 4.5-30mmHg) with a bath temperature of up to 60 °C;
(iii) in general, the course of reactions was followed by TLC or MS and reaction times are given for illustration only;
(iv) final products had satisfactory proton nuclear magnetic resonance (NMR) spectra and/or mass spectral data;
(v) yields are given for illustration only and are not necessarily those which can be obtained by diligent process development; preparations were repeated if more material was required;
(vii) when given, NMR data is in the form of delta values for major diagnostic protons, given in parts per million (ppm) relative to tetramethylsilane (TMS) as an internal standard, determined at 400 MHz using deuterated chloroform (CDCl₃) as solvent unless otherwise indicated;
(vii) chemical symbols have their usual meanings; SI units and symbols are used;
(viii) solvent ratios are given in volume:volume (v/v) terms; and
(ix) mass spectra were run with an electron energy of 70 electron volts in the chemical ionization (CI) mode using a direct exposure probe; where indicated ionization was effected by electron impact (EI), fast atom bombardment (FAB); electrospray (ESP); or atmospheric pressure chemical ionisation (APCI); values for m/z are given; generally, only ions which indicate the parent mass are reported;
(x) where a synthesis is described as being analogous to that described in a previous example the amounts used are the millimolar ratio equivalents to those used in the previous example;
(xi) the following abbreviations have been used:

| | |
|---|---|
| THF | tetrahydrofuran; |
| DMF | *N,N*-dimethylformamide; |
| EtOAc | ethyl acetate; |
| AcOH | acetic acid; |
| DCM | dichloromethane; and |
| DMSO | dimethylsulphoxide; and |

(xii) a Vigreux column is a glass tube with a series of indentations such that alternate sets of indentations point downward at an angle of 45 degree in order to promote the redistribution of liquid from the walls to the center of the column; The Vigreux column used herein is 150 mm long (between indents) with a 20 mm diameter and it was manufactured by Lab Glass.

### METHOD 1

### 2-(1-Ethoxy-ethylidene)-malononitrile

Triethyl oithoacetate (97 g, 0.6 mol), malononitrile (33 g, 0.5 mol) and glacial acetic acid (1.5 g) were placed in a 1L flask equipped with a stirrer, thermometer and a Vigreux column (20 x 1 in.) on top of which a distillation condenser was placed. The reaction mixture was heated and ethyl alcohol began to distill when the temperature of the reaction mixture was about 35-90 °C. After about 40 min., the temperature of the reaction mixture reached 140 °C. Then the reaction was concentrated in a rotary evaporator to remove the low-boiling materials and the residue was crystallized from absolute alcohol to yield the pure product (62.2 g, 91%) as a light yellow solid mp 91.6 °C.

### METHOD 2

### (2E)-2-Cyano-3-ethoxybut-2-enethioamide

2-(1-Ethoxy-ethylidene)-malononitrile (method 1) (62 g, 0.45 mol) was dissolved in anhydrous benzene (800 mL) and 1 mL of triethylamine was added as catalyst. The mixture was stirred and hydrogen sulfide was bubbled into this solution for 40 min and a solid formed. The precipitated solid was filtered off and dried. The solid was recrystallized from absolute alcohol (100 mL) filtered and dried to isolate the pure (2*E*)-2-cyano-3-ethoxybut-2-enethioamide (19.3 g, 25%) as light brown crystals.

### METHOD 3

### (2E)-3-Amino-2-cyanobut-2-enethioamide

(2E)-2-cyano-3-ethoxybut-3-enethioamide (method 2) (19.2 g, 0.136 mol) was dissolved in a saturated solution of ammonia in methanol (500 mL) and stirred at r.t. overnight. The reaction mixture was concentrated and the residue was dissolved in hot water (600 mL) and the undissoved solid was filtered and dried to recover 6 g of the starting thiocrotonamide. The aqueous solution on standing overnight provided the pure (2*E*)-3-amino-2-cyanobut-2-enethioamide (6.85 g, 63%) as off-white crystals. Having the following properties ¹H NMR (300 MHz, DMSO-d₆) δ 2.22 (s, 3H), 7.73 (bs, 1H), 8.53 (bs, 1H), 9.01 (bs, 1H), 11.60 (bs, 1H).

### METHOD 4

### 5-Amino-3-methylisothiazole-4-carbonitrile

To a stirred solution of (2*E*)-3-amino-2-cyanobut-2-enethioamide (method 3) (6.83 g, 48.4 mmol) in methanol (300 mL) was added dropwise 13.6 mL (124 mmoL) of 30% hydrogen peroxide. The mixture was stirred at 60 °C for 4 h and evaporated to 60 mL in a rotary evaporator and cooled in an ice-bath. The crystallized product was filtered off and recrystallized from EtOAc to provide the pure product 5-amino-3-methylisothiazole-4-carbonitrile (5.41 g. 80%) as a white crystalline solid. Having the following properties ¹H NMR (300 MHz, DMSO-d₆) δ 2.24 (s, 3H), 8.00 (bs, 2H).

### METHOD 5

### N-(4-Cyano-3-methyl-isothiazol-5-yl)-butyramide

To a solution of 5-amino-3-methylisothiazole-4-carbonitrile (method 4) (5.31 g, 38.2 mmol) in DCM (200 mL) at 0 °C, NEt₃ (5 g, 50 mmol) was added followed by the dropwise addition of a solution of the butyryl chloride (4.88 g, 45.8 mmol) in DCM (50 mL). After the completion of the addition the reaction mixture was allowed to warm to r.t. and stirred overnight. The reaction mixture was washed with water (100 mL), 1N HCl (100 mL), brine (200 mL) and dried over Na₂SO₄. Concentration of the DCM layer provided the crude product which was triturated from DCM/hexanes (1/10) and filtered off to isolate the pure N-(4-cyano-3-methyl-isothiazol-5-yl)-butyramide (7.57 g, 95%) as an orange solid.

### METHOD 6

### 5-Butyrylamino-3-methyl-isothiazole-4-carboxylic acid amide

To a solution of N-(4-cyano-3-methyl-isothiazol-5-yl)-butyramide (method 5) (4.18 g, 20 mmol) in 30% aqueous NH₄OH (250 mL), was added dropwise 100 mL, of hydrogen peroxide at r.t. After the completion of the addition the reaction mixture was stirred at 60 °C overnight after which the TLC showed the complete disappearance of SM. The reaction mixture was cooled and extracted with chloroform (3 x 100 mL). The organic layer was dried (Na₂SO₄) and concentrated to get the pure 5-butyrylamino-3-methyl-isothiazole-4-carboxylic acid amide (2.9 g, 72%) as a white solid. Having the following properties ¹H NMR (300 MHz) δ 1.03 (t, 3H), 1.79 (m, 2H), 2.54 (t, 3H), 2.69 (s, 3H), 5.97 (bs, 2H), 11.78 (bs, 1H).

### METHOD 7

### 3-Methyl-6-propyl-5H-isothiazolo[5,4-d]pyrimidin-4-one

5-Butyrylamino-3-methyl-isothiazole-4-carboxylic acid amide (method 6) (1.9 g, 8.3 mmol) was suspended in 75 mL of 30% NH₃ and then was heated to 140 °C for 4h in a pressure reactor. The mixture was cooled and neutralized to pH δ. The precipitated 3-methyl-6-propyl-5H isothiazolo[5,4-d]pyrimidin-4-one was filtered off, washed with water (100 mL) and dried in vacuum oven at 40 °C overnight to get 800 mg (34%) of pure product. Having the following properties ¹H NMR (300MHz) δ 1.03 (t, 3H), 1.74 (m, 2H), 2.67 (t, 3H), 2.78 (s, 3H).

### METHOD 8

### 5-(3-Fluoro-benzyl)-3-methyl-6-propyl-5H-isothiazolo[5,4-d]pyrimidin-4-one

To a solution of 3-methyl-6-propyl-5*H*-isothiazolo[5,4-d]pyrimidin-4-one (method 7) (2.09g, 10 mmol) in 20 mL of anhydrous DMF was added anhydrous K₂CO₃ (2.76 g, 20 mmol) followed by 3-fluorobenzyl bromide (2.79g, 15 mmol) and the mixture was stirred at room temperature overnight. Solvents were removed by evaporation. The residue obtained was triturated with water (60 mL) and stirred for 30 minutes. The solid separated was collected by filtration and subsequently purified by crystallization from a mixture of EtOAc and hexanes (1:5) and dried. Yield 1.78g (56%). Having the following properties ¹H NMR (300 MHz, DMSO-d₆). δ:0.87 (t, 3H), 1.65-1.67 (m, 2H), 2.73 (s, 3H), 2.75 (t, 2H), 5.39 (s, 2H), 7.04 (d, 1H). 7.05-7.09 (m, 2H), 7.13-7.38 (m, 1H).

### METHOD 9

### 6-(1-Bromo-propyl)-5-(3-fluoro-benzyl)-3-methyl-5H-isothiazolo[5.4-d]pyrimidin-4-one

To a solution of 5-(3-fluoro-benzyl)-3-methyl-6-propyl-5H-isothiazolo[5,4-d]pyrimidin-4-one (method 8) (1.78g, 5.62 mmol) and sodium acetate (4.6g, 56.2 mmol) in acetic acid (40 mL) at 100 °C, a solution of the bromine (1.8g, 11.24 mmol) in acetic acid (10 mL) was added dropwise over a period of 30 minutes. The mixture was stirred for additional 15 minutes and cooled to 25 °C. The solvents were removed by evaporation and the residue was dissolved in EtOAc (100 mL) and washed with 100 mL each of water, 10 % sodium thiosulfate solution and brine. Solvents were removed by evaporation and the residue was purified by column chromatography on silica, eluting with 10-15% of EtOAc in hexanes. Yield 890 mg (41%). Having the following properties ¹H NMR (300 MHz, DMSO-d₆) δ 0.87 (t, 3H), 2.05-2.20 (m, 1H), 2.30-2.40 (m, 1H), 2.70 (s, 3H), 5.07 (t, 1H), 5.27 (d, 1H), 5.66 (d, 1H), 7.05-7.25 (m, 3H), 7.3 8-7.40 (m, 1H).

### METHOD 10

### (3-{1-[5-(3-Fluoro-benzyl)-3-methyl-4-oxo-4,5-dihydro-isothiazolo[5.4-d]pyrimidin-6-yl]-propylamino}-propyl)-carbamic acid tert-butyl ester

To a suspension of 6-(1-bromo-propyl)-5-(3-fluoro-benzyl)-3-methyl-5H-isotluazolo[5,4-d]pyrimidin-4-one (method 9) (90mg, 2.25 mmol) in DMF (10mL) was added (3-amino-propyl)-carbamic acid tert-butyl ester (700mg, 4.02 mmol) and diisopropyl ethyl amine (740mg, 5.74 mmol, 1mL). The mixture was then stirred for 30 minutes. It was diluted with EtOAc (100 mL) and washed with water (2x100 mL). The EtOAc layer was then dried over MgSO₄ and evaporated to dryness. The product was used in the next step without purification. Having the following properties *m*/*z* 490 (MH⁺).

### METHOD 11

### {3-[{[-5-(3-Fluoro-benzyl)-3-methyl-4-oxo-4.5-dihydro-isothiazolo[5,4-d]pyrimidin-6-yl]-propyl}-(4-methyl-benzoyl)-amino]-propyl}-carbamic acid tert-butyl ester

To a solution of (3-{1-[5-(3-fluoro-benzyl)-3-methyl-4-oxo-4,5-dihydro-isothiazolo[5,4-d]pyrimidin-6-yl]-propylamino}-propyl)-carbamic acid tert-butyl ester (method 10) (amount isolated from method 10 used) in chloroform (20 mL) was added diisopropyl ethyl amine (740mg, 5.74 mmol, 1 mL). The reaction mixture was brought to 50 °C and a solution of p-toluoyl chloride (521mg, 3.35 mmol) in chloroform (5 mL) was added dropwise. The mixture was maintained at the same temperature for 2h and then at 25 °C for 18h and subsequently diluted with chloroform and washed with water (2X25 mL). The organic layer was evaporated to dryness and the residue was purified by column chromatography on silica, eluting with 15% EtOAc in hexane gave the product. Yield 590 mg (4 3%). Having the following properties *m*/*z* 608 (MH⁺); ¹H NMR (300 MHz, DMSO-d₆, 95°C) δ0.70 (t, 3H), 1.10-1.15 (m, 1H), 1.26(s,9H), 1.29-1.37 (m, 1H), 1.89-1.90 (m, 1H), 2.32-2.47 (m, 1H), 2.46 (s, 3H), 2.47-2.49 (m, 2H), 2.71 (s, 3H), 3.22-3.25 (m, 2H), 4.99 (d, 1H), 5.59 (bis, 1H), 5.73 (d, 1H), 6.03 (t, 1H): 6.96-7.18 (m, 3H), 7.18-7.20 (m, 4H), 7.20-7.22 (m, 1H).

### METHOD 12

### N-(4-Cyano-3-methyl-isothiazol-5-yl)-3-methyl-butyramide

To a solution of 5-amino-3-methyl-isothiazole-4-carbonitrile (method 4) (6.38 g, 45.9 mmol) in pyridine (20 mL) at 0 °C, isovaleryl chloride (6.65 g, 55 mmol) was added dropwise. After the completion of the addition the reaction mixture was allowed to warm to r.t. and stirred overnight. The TLC and the MS showed the complete disappearance of the starting material and the reaction mixture was diluted with CHCl₃ (200 mL), washed with water (200,mL), 2N HCl (225 mL), satd. NaHCO₃ (200 mL), brine (200 mL) and dried over Na₂SO₄. Concentration of the CHCl₃ layer provided the crude product which was triturated from DCM/hexanes (1/10) and filtered off to isolate N-(4-cyano-3-methyl-isothiazol-5-yl)-3-methyl-butyramide (8.1 g, 79%) as an off-white crystalline solid. Having the following properties ¹H NMR (300 MHz) δ 1.04 (d, 6H), 2.18-2.32 (m, 1H), 2.46 (d, 2H), 2.53 (s, 3H), 9.87 (bs, 1H).

### METHOD 13

### 3-Methyl-5-(3-methyl-butyrylamino)-isothiazole-4-carboxylic acid amide

To a solution of N-(4-cyano-3-methyl-isothiazol-5-yl)-3-methyl-butyramide (method 12) (8 g, 35.8 mmol) in 30% aqueous NH₄OH (200 mL), was added dropwise 100 mL of hydrogen peroxide at r.t. After the completion of the addition the reaction mixture was stirred at 60 °C overnight after which the TLC showed the complete disappearance of SM. The reaction mixture was concentrated to 40 mL and extracted with chloroform (3 x 100 mL). The organic layer was dried (Na₂SO₄) and concentrated to obtain 3-methyl-5-(3-methyl-butyrylamino)-isothiazole-4-carboxylic acid amide (6.1 g, 71%) as a light yellow solid. Having the following properties ¹H NMR (300 MHz) δ 1.03 (d, 6H), 2.24 (m, 1H), 2.43 (d, 2H), 2.69 (s, 3H), 5.98 (bs, 2H), 11.77 (bs, 1H).

### METHOD 14

### 6-Isobutyl-3-methyl-5H-isothiazolo[5,4-d]pyrimidin-4-one

3-Methyl-5-(3-methyl-butyrylamino)-isothiazole-4-carboxylic acid amide (method 13) (6 g, 25 mmol) was suspended in 150 mL of 30% NH₃ and then was heated to 140 °C for 5h in a pressure reactor. The mixture was cooled and neutralized to pH 7. The reaction mixture was extracted with EtOAc (3 x 100 mL) and the combined organic layers were washed with water (100 mL), brine (100 mL) and concentrated to get the crude product which was further purified by column (silica gel) chromatography using 30% EtOAc in hexanes as eluent. Concentration of the pure product fractions provided 6-isobutyl-3-methyl-5H-isothiazolo[5,4-d]pyrimidin-4-one (2.2 g, 38%) as an off-white powder. Having the following properties ¹H NMR (300 MHz) δ z (d, 6H), 2.32 (m, 1H), 2.69 (d, 2H), 2.82 (s, 3H).

### METHOD 15

### 5-Benzyl-6-isobutyl-3-methyl-5H-isothiazolo[5,4-d]pyrimidin-4-one

To a solution of 6-isobutyl-3-methyl-5H-isothiazolo[5,4-d]pyrimidin-4-one (method 14) (1.31 g, 5.8 mmol) in 20 mL of anhydrous DMF was added 1.38 g (10 mmol) of anhydrous K₂CO₃ followed by benzyl bromide (1.18 g, 6.9 mmol) and the mixture was stirred at room temperature overnight. The TLC of the reaction mixture showed the complete disappearance of the SM. The reaction mixture was poured into ice-cold water and extracted with EtOAc (3X100 mL). The combined extracts were washed with water (100 mL), brine (100 mL), dried (Na₂SO₄) and concentrated. The TLC and the ¹H NMR showed the presence of two products (*N* alkylated as well as *O*-alkylated products) in a ratio of 7:3. The products were separated by column (silica gel, 116 g) chromatography using 10% EtOAc in hexanes. 5-Benzyl-6-isobutyl-3-methyl-5H-isothiazolo[5,4-d]pyrimidin-4-one was isolated as white crystalline solid (1.3 g, 70%). Having the following properties m/z 314 (MH⁺), ¹H NMR (300 MHz) δ 0.94 (d, 6H), 2.23-2.37 (m, 1H), 2.64 (d, 2H), 2.82 (s, 3H), 5.38 (s, 2H), 7.10-7.38 (m, 5H).

### METHOD 15a

The following compounds were synthesized according to Method 15:

| **Method #** | **Compound Name** | **m/z** | **Alkylating agent** |
|---|---|---|---|
| 15a | 5-(3-Fluoro-benzyl)-6-isobutyl)-3-methyl-5H-isothiazolo[5,4-d]pyrinidin-4-one | 332 (MH⁺) | 3-fluorobenzyl bromide |

### METHOD 16

### 5-Benzyl-6-(1-bromo-2-methyl-propyl)-3-methyl-5H-isothiazolo[5,4-d]pyrimidin-4-one

To a solution of 5-benzyl-6-isobutyl-3-methyl-5H-isothiazolo[5,4-d]pyrimidin-4-one (method 15) (1.3 g, 4.2 mmol) and sodium acetate (2 g) in acetic acid (10 mL) at 100 °C, a solution of the bromine (1.32 g, 8.4 mmol) in acetic acid (10 mL) was added dropwise over a period of 20 minutes. The reaction mixture was stirred at that temperature for 30 min and cooled and the TLC (eluent 10% EtOAc in hexanes) and MS showed the complete disappearance of the SM and only the product. The reaction mixture was poured into ice water and extracted with EtOAc (3X 60 mL) and the organic layers were combined and washed with 2% sodium thiosulfate solution (60 mL), water (100 mL), brine (100 mL) and dried over Na₂SO₄. Concentration of the organic layer provided 5-benzyl-6-(1-bromo-2-methyl-propyl)-3-methyl-5H-isothiazolo[5,4-d]pyrimidin-4-one (1.61 g, 99%) as white crystalline solid. Having the following properties *m*/*z* 392, 394 (MH⁺), ¹H NMR (300 MHz) δ 0.54 (d, 3H), 1.11 (d, 3H), 2.62-2.76 (m, 1H), 2.83 (s, 3H), 4.42 (d, 1H), 4.80 (d, 1H), 6.22 (d, 1H), 7.12-7.43 (m, 5H).

### METHOD 16a

The following compounds were synthesized according to Method 16 starting from 5-(3-fluoro-benzyl)-6-isobutyl-3-methyl-5H-isothiazolo[5,4-d]pyrimidin-4-one (Method 15a):

| **Method #** | **Compound Name** | **m/z** |
|---|---|---|
| **16a** | 6-(1-Bromo-2-methyl-propyl)-5-(3-fluoro-benzyl)-3-methyl-5H-isothiazolo[5,4-d]pyrimidin-4-one | 410, 412 (MH⁺) |

### METHOD 17

### 6-(1-Azido-2-methyl-propyl)-5-benzyl-3-methyl-5H-isothiazolo[5,4-d]pyrimidin-4-one

To a solution of 5-benzyl-6-(1-bromo-2-methyl-propyl)-3-methyl-5H-isothiazolo[5,4-d]pyrimidin-4-one (method 16) (0.6 g, 1.52 mmol) in anhydrous DMF (20 mL), sodium azide (0.65 g, 10 mmol) was added and the mixture was stirred at room temperature for 1 hour. The TLC of the RM showed the complete disappearance of the starting bromide. The reaction mixture was poured into ice water (300 mL) and extracted with EtOAc (3 X 100 mL). The organic layer was washed with water (100 mL), brine (100 mL) and dried (Na₂SO₄)- Concentration of the organic layer provided the crude product which was purified by column (silica gel) chromatography using 30% EtOAc in hexanes as eluent to isolate 6-(1-azido-2-methyl-propyl)-5-benzyl-3-methyl-5H-isothiazolo[5,4-d]pyrimidin-4-one (0.506 g, 94%) as a low melting solid. Having the following properties *m*/*z* 355 (MH⁺), ¹H NMR (300 MHz) δ 0.57 (d, 3H), 1.07 (d, 3H), 2.50-2.74 (m, 1H), 2.98 (s, 3H), 3.71 (d, 1H), 5.05 (d, 1H), 5.78 (d, 1H), 7.12-7.40 (m, 5H).

### METHOD 17a

The following compounds were synthesized according to Method 17 starting from 6-(1-bromo-2-methyl-propyl)-5-(3-fluoro-benzyl)-3-methyl-5H-isothiazolo[5,4-d]pyrimidin-4-one (Method 16a):

| **Method #** | **Compound Name** | **m/z** |
|---|---|---|
| **17a** | 6-(1-Azido-2-methyl-propyl)-5-(3-fluoro-benzyl)-3-methyl-5H-isothiazolo[5,4-d]pyrimidin-4-one | 373 (MH⁺) |

### METHOD 18

### 6-(1-Amino-2-methyl-propyl)-5-benzyl-3-methyl-5H-isothiazolo[5,4-d]pyrimidin-4-one

To a solution of 6-(1-azido-2-methyl-propyl)-5-benzyl-3-methyl-5H-isothiazolo[5,4-d]pyrimidin-4-one (method 17) (0.5 g, 1.41 mmol) in methanol (20 mL) was added 5% Pd/C (20% by wt.) and the resulting mixture was stirred at r.t. in an atmosphere of H₂ and the progress of the reaction was monitored by MS. After the disappearance of the starting material the reaction mixture was filtered through celite and washed with EtOAc. Concentration of the filtrate provided 6-(1-amino-2-methyl-propyl)-5-benzyl-3-methyl-5H-isothiazolo[5,4-d]pyrimidin-4-one as a thick oil. The product was used as such in the next reaction with out further purification. Having the following properties *m*/*z* 349 (MH⁺).

### METHOD 18a

The following compounds were synthesized according to Method 18 starting from 6-(1-azido-2-methyl-propyl)-5-(3-fluoro-benzyl)-3-methyl-5H-isothiazolo[5,4-d]pyrimidin-4-one (Method 17a):

| **Method #** | **Compound Name** | **m/z** |
|---|---|---|
| **18a** | 6-(1-Amino-2-methyl-propyl)-5-(3-fluoro-benzyl)-3-methyl-5H-isothiazolo[5,4-d]pyrimidin-4-one | 367 (MH⁺) |

### METHOD 19

### {2-[1-(5-Benzyl-3-methyl-4-oxo-4,5-dihydro-isothiazolo[5,4-d]pyrimidin-6-yl)-2-methyl-propylamino]-ethyl}-carbamic acid tert-butyl ester

To a mixture of 6-(1-amino-2-methyl-propyl)-5-benzyl-3-methyl-5H-isothiazolo[5,4-d]pyrimidin-4-one (method 18) (1.1 g, 3.35 mmol) and molecular sieves (4 A, 20 g) in DCM was added the solution of (2-oxo-ethyl)-carbamic acid tert-butyl ester (0.53 g, 3.35 mmol). The resulting reaction mixture was stirred at rt for 7 h. After addition of AcOH (2 drops), sodium triacetoxy borohydride (0.71 g, 3.35 mmol) was added. The reaction mixture was stirred overnight at rt. It was filtered through a pad of celite and celite cake was washed with DCM. The filtrate was washed with sat. NaHCO₃ (15 ml) and org. layer was separated. Aq. layer was re-extracted with DCM (100 mL). The combined org. layers were dried over MgSO4, filtered and concentrated in vacuo to yield {2-[1-(5-Benzyl-3-methyl-4-oxo-4,5-dihydro-isothiazolo[5,4-d]pyrimidin-6-yl)-2-methyl-propylamino]-ethyl-carbamic acid tert-butyl ester (1.50 g, white foam). The crude product was used in next step. Having the following properties *m*/*z* 472 (MH⁺).

### METHODS 19a-19b

The following compounds were synthesized according to Method 19:

| **Method #** | **Compound Name** | **m/z** | **S M** |
|---|---|---|---|
| **19a** | (3-{1-[5-(3-Fluoro-benzyl)-3-methyl-4-oxo-4,5-dihydro-isothiazolo[5,4-d]pyrimidin-6-yl]-2-methyl-propylamino} -propyl)-carbamic acid tert-butyl ester | 504 (MH⁺) | (3-oxo-propyl)-carbamic acid tert-butyl ester and Method 18a |
| **19b** | {3-[1-(5-Benzyl-3-methyl-4-oxo-4,5-dihydro-isothiazolo[5,4-d]pyrimidin-6-yl)-2-methylpropylamino]-propyl}-carbantic acid tert-butyl ester | 486 (MH⁺) | (3-oxo-propyl)-carbamic acid tert-butyl ester and Method 18 |

### METHOD 20

### 5-Benzyl-6-[1-[1,3]dioxolan-2-yl-ethylamino)-2-methyl-propyl]-3-methyl-5H-isothiazolo[5,4-d]pyrimidin-4-one

To a solution of 6-(1-amino-2-methyl-propyl)-5-benzyl-3-methyl-5H-isothiazolo[5,4-d]pyrimidin-4-one (method 18) (1.6 g, 4.88 mmol) in anhydrous DMF (20 mL), 2-(2-bromoethyl)-[1,3]dioxolane (0.88 g, 4.88 mmol) was added and the resulting solution was heated at 70 °C for 2 h. The reaction mixture was cooled, diluted with water and extracted with EtOAc (3 x 60 mL). The combined organic extracts were dried (Na₂SO₄) and concentrated to provide the crude product (2 g), which was used as such in the next reaction. Having the following properties *m*/*z* 429 (MH⁺); ¹H-NMR (300 MHz) δ 0.88 (d, 3H), 0.96 (d, 3H), 54-1.62 (m, 2H), 1.86-2.05 (m, 2H), 2.18 (bs, 1H), 2.38-2.46 (m, 1H), 2.84 (s, 3H), 3.57 (d, 1H), 3.74-3.94 (m, 4H), 4.78 (t, 1H), 4.99 (d, 1H), 5.8 (d, 1H), 7.15-7.38 (m, 5H).

### METHOD 21

### {2-[[1-(5-Benzyl-3-methyl-4-oxo-4,5-dihydro-isothiazolo[5,4-d]pyrimidin-6-yl)-2-methylpropyl]-(4-methyl-benzoyl)-amino]-ethyl}-carbamic acid tert-butyl ester

To a solution of {2-[1-(5-benzyl-3-methyl-4-oxo-4,5-dihydro-isothiazolo[5,4-d]pyrimidin-6-yl)-2-methyl-propylamino]-ethyl}-carbamic acid tert-butyl ester (method 19) (1.50 g, 3.20 mmol), DIEA (0.75 g, 5.8 mmol) in CHCl₃ (30 mL) at 60 °C under nitrogen atmosphere was added a solution of p-toluoyl chloride (0.74g, 4.8 mmol) in CHCl₃ (60 mL). The reaction mixture was refluxed for 27 h and then cooled to rt. The reaction mixture was treated with sat. NaHCO₃ (50 ml). The organic layer was separated and the aqueous layer was re-extracted with CHCl₃ (150 mL). The combined org. layers were dried over MgSO₄, filtered and concentrated in vacuo to yield {2-[[1-(5-Benzyl-3-methyl-4-oxo-4,5-dihydro-isothiazolo[5,4-d]pyrimidin-6-yl)-2-methyl-propyl]-(4-methyl-benzoyl)-amino]-ethyl}-carbamic acid tert-butyl ester (1.10 g, 69% overall yield). m/z 590 (MH⁺).

### METHODS 21a-21h

The following compounds were synthesized according to Method 21:

| **Method #** | **Compound Name** | **m/z** | **SM** | **Acylating agent** |
|---|---|---|---|---|
| **21a** | {3-[{1-[5-(3-Fluoro-benzyl)-3-methyl-4-oxo-4,5-dihydro-isothiazolo[5,4-d]pyrimidin-6-yl]-2-methyl-propyl} -(4-methyl-benzoyl)-amino]-propyl }-carbamic acid tert-butyl ester | 622 (MH⁺) | Method 19a | 4-methyl-benzoyl chloride |
| **21b** | {2-[[1-(5-Benzyl-3-methyl-4-oxo-4,5-dihydro-isothiazolo[5,4-d]pyrimidin-6-yl)-2-methyl-propyl]-(4-bromo-benzoyl)-amino]-ethyl}-carbamic acid tert-butyl ester | 654, 656 (MH⁺) | Method 19 | 4-bromo-benzoyl chloride |
| **21c** | {2-[[1-(5-Benzyl-3-methyl-4-oxo-4,5-dihydro-isothiazolo[5,4-d]pyrimidin-6-yl)-2-methyl-propyl]-(3-fluoro-4-methyl-benzoyl)-amino]-ethyl} -carbamic acid tert-butyl ester | 608 (MH⁺) | Method 19 | 3-fluoro-4-methylbenzoyl chloride |
| **21d** | {3-[[1-(5-Benzyl-3-methyl-4-oxo-4,5-dihydro-isothiazolo[5,4-d]pyrimidin-6-yl)-2-methyl-propyl]-(3-fluoro-4-methyl-benzoyl)-amino]-propyl}-carbamic acid tert-butyl ester | 622 (MH⁺) | Method 19b | 3-fluoro-4-methylbenzoyl chloride |
| **21e** | {3-[[1-(5-Benzyl-3-methyl-4-oxo-4,5-dihydro-isothiazolo[5,4-d]pyrimidin-6-yl)-2-methyl-propyl]-(4-bromo-benzoyl)-amino]-propyl}-carbamic acid tert-butyl ester | 668, 670 (MH⁺) | Method 19b | 4-bromo-benzoyl chloride |
| **21f** | N-[1-(5-Benzyl-3-methyl-4-oxo-4,5-dihydro-isothiazolo[5,4-d]pyrimidin-6-yl)-2-methyl-propyl]-N-(2-[1,3]dioxolan-2-yl-ethyl)-4-methyl-benzamide | 547 (MH⁺) | Method 20 | 4-methyl-benzoyl chloride |
| **21g** | N-[1-(5-Benzyl-3-methyl-4-oxo-4,5-dihydro-isothiazolo[5,4-d]pyrimidin-6-yl)-2-methyl-propyl]-4-bromo-N-(2-[1,3]dioxolan-2-yl-ethyl)-benzamide | 611, 613 (MH⁺) | Method 20 | 4-bromo-benzoyl chloride |
| **21h** | N-[1-(5-Benzyl-3-methyl-4-oxo-4,5-dihydro-isothiazolo[5,4-d]pyrimidin-6-yl)-2-methyl-propyl]-N-(2-[1,3]dioxolan-2-yl-ethyl)-3-fluoro-4-methyl-benzamide | 565 (MH⁺) | Method 20 | 3-fluoro-4-methyl-benzoyl chloride |

### METHOD 22

### N-[1-(5-Benzyl-3-methyl-4-oxo-4,5-dihydro-isothiazolo[5,4-d]pyrimidin-6-yl)-2-methyl-propyl]-4-bromo-N-(3-oxo-propyl)-benzamide

N-[1-(5-Benzyl-3-methyl-4-oxo-4,5-dihydro-isothiazolo[5,4-d]pyrimidin-6-yl)-2-methylpropyl]-4-bromo-N-(2-[1,3]dioxolan-2-yl-ethyl)-benzamide (method 21g) (1.1 g, 1.8 mmol) was dissolved in 20 mL of 80% acetic acid and the solution was heated at 80 °C for 2h. The reaction mixture was cooled in an ice bath and neutralized slowly by the addition of solid NaHCO₃ until pH 8. The thus obtained mixture was extracted with DCM (3 x 100 mL). The combined organic layers was washed with brine (100 mL) and dried (Na₂SO₄). Concentration of the DCM layer provided a yellow foam (1 g crude yield) and it was used as such in the next reaction. *m*/*z* 567, 569 (MH⁺).

### METHODS 22a-22b

The following compounds were synthesized according to Method 22:

| **Method #** | **Compound Name** | **m/z** | **SM** |
|---|---|---|---|
| **22a** | N-[1-(5-Benzyl-3-methyl-4-oxo-4,5-dihydroisothiazolo[5,4-d]pyrimidin-6-yl)-2-methyl-propyl]-4-methyl-N-(3-oxo-propyl)-benzamide | 503 (MH⁺) | Method 21f |
| **22b** | N-[1-(5-Benzyl-3-methyl-4-oxo-4,5-dihydroisothiazolo[5,4-d]pyrimidin-6-yl)-2-methyl-propyl]-3-fluoro-4-methyl-N-(3-oxo-propyl)-benzamide | 521 (MH⁺) | Method 21h |

### METHOD 23

### 3-Methyl-5-(3-methyl-butyryl)-isoxazole-4-carboxylic acid amide

A mixture of 5-amino-3-methyl-isoxazole-4-carboxylic acid amide (10 g, 70 mmol) in 25 ml of isovaleric anhydride was stirred at 110-145°C for 1h. The brown solution was diluted with hexane (500 ml) and cooled down. The precipitated gum was separated from the mixture and washed with hexane, dried in vacuo. 3-Methyl-5-(3-methyl-butyryl)-isoxazole-4-carboxylic acid amide was obtained as a yellow gum. Further used without purification in method 24.

### METHOD 24

### 6-Isobutyl-3-methyl-5H-isoxazolo[5,4-d]pyrimidin-4-one

A suspension of 3-methyl-5-(3-methyl-butyryl)-isoxazole-4-carboxylic acid amide (method 23) (split into 40 vials) in 3.5 ml of 2N NaOH aq was subjected to microwave irradiation at 140°C for 20min. The resulting solution was cooled with an ice bath, and the pH was adjusted to 1~3 with concentrated HCl. The solid was filtered, washed with water, dried over vacuum at 40°C overnight. 6-Isobutyl-3-methyl -5H-isoxazolo[5,4-d]pyrimidin-4-one (8 g) was obtained as a white solid. 55% yield for two steps. Having the following properties *m*/*z*: 208 (MH⁺), ¹H NMR (DMSO-d₆): 0.76 (d, 6H), 1.95 (m, 1H), 2.25 (s, 3H), 2.32 (d, 2H), 12.55 (s, 1H).

### METHOD 25

### 5-(3-Fluoro-benzyl)-6-isobutyl-3-methyl-5H-isoxazolo[5,4-d]pyrimidin-4-one

A suspension of 6-isobutyl-3-methyl-5H-isoxazolo[5,4-d]pyrimidin-4-one (method 24) (1.24 g, 6.0 mmol), 3-fluorobenzylbromide (1.13 g, 6.0 mmol), potassium carbonate (1.38 g, 10.0 mmol) in 20 ml DMF was stirred at room temperature for 2 days. The mixture was diluted with water, extracted with EtOAc (100 ml x 3), the combined organic phases were dried, concentrated, purified by flash column chromatography (elute: hexane- EtOAc = 10:3). 5-(3-Fluoro-benzyl)-6-isobutyl-3-methyl-5H-isoxazolo[5,4-d]pyrimidin-4-one was obtained as white solid (1.0 g, 3.17 mmol) (53%). Having the following properties *m*/*z*: 316 (MH⁺), 1H-NMR (300 MHz) δ : 0.96 (d, 6H), 2.27-2.41 (heptet, 1H), 2.59 (s, 3H), 2.65 (d, 2H), 5.37 (s, 2H), 6.50-7.05 (m, 3H), 7.30-7.40 (m, 1H).

### METHOD 26

### 6-(1-Bromo-2-methyl-propyl)-5-(3-fluoro-benzyl)-3-methyl-5H-isoxazolo[5,4-d]pyrimidin-4-one

A solution of 5-(3-fluoro-benzyl)-6-isobutyl-3-methyl-5H-isoxazolo[5,4-d]pyrimidin-4-one (method 25) (1.0g, 3.17 mmol) and sodium acetate (1.0g, 12.1 mmol) in glacial acetic acid (20 ml) was treated with a preformed bromine solution (1.0g bromine in 20 ml of glacial acetic acid) (0.32 ml, 6.29 mmol). The mixture was stirred at 110-120°C. for 1 day. Water was added to the mixture to which was subsequently added potassium carbonate and extracted with DCM (20 ml x 3), the combined organic phases were washed with water and dried, then concentrated to give the crude product which was purified by ISCO (elute: hexane- EtOAc). 6-(1-Bromo-2-methyl-propyl)-5-(3-fluoro-benzyl)-3-methyl-5H-isoxazolo[5,4-d]pyrimidin-4-one was obtained as a yellow gum (1.1g, 2.79mmol) (88%). Having the following properties *m*/*z*: 394, 396 (MH⁺), 1H-NMR (300 MHz) δ : 0.61 (d, 3H), 1.14 (d, 3H), 2.64 (s, 3H), 2.71-2.80 (m, 1H), 4.35 (d, 2H), 4.82 (d, 1H), 6.13 (d, 1H), 6.82-7.03 (m, 3H), 7.32-7.39 (m, 1H).

### METHOD 27

### 6-(1-Azido-2-methyl-propyl)-5-(3-fluoro-benzyl)-3-methyl-5H-isoxazolo[5,4-d]pyrimidin-4-one

A suspension of 6-(1-bromo-2-methyl-propyl)-5-(3-fluoro-benzyl)-3-methyl-5H-isoxazolo[5,4-d]pyrimidin-4-one (method 26) (1.10g, 2.79 mmol) and sodium azide (0.88g, 13.9 mmol, 5 eq.) in DMF (10 ml) was stirred at 60°C for 1h. Water (10 ml) was added to the mixture and then extracted with EtOAc (3 × 20 ml). The combined organic phases were washed with brine (20 ml), dried, concentrated and purified by ISCO (Hexane- EtOAc). 6-(1-Azido-2-methylpropyl)-5-(3-fluoro-benzyl)-3-methyl-5H-isoxazolo[5,4-d]pyrimidin-4-one was obtained (0.98 g, 2.72mmol (97%) as a colourless oil. Having the following properties *m*/*z*: 357 (MH⁺), 1H-NMR (300 MHz) δ: 0.59 (d, 3H), 1.10 (d, 3H), 2.62 (s, 3H), 2.58-2.70 (m, 1H), 3.65 (d, 2H), 5.05 (d, 1H), 5.75 (d, 1H), 6.82-7.03 (m, 3H), 7.31-7.39 (m, 1H).

### METHOD 28

### 6-(1-Amino-2-methyl-propyl)-5-(3-fluoro-benzyl)-3-methyl-5H-isoxazolo[5,4-d]pyrimidin-4-one

A mixture of 6-(1-azido-2-methyl-propyl)-5-(3-fluoro-benzyl)-3-methyl-5H-isoxazolo[5,4-d]pyrimidin-4-one (method 27) (0.9g, 2.72 mmol), triphenylphosphine (0.78g, 3.0 mmol) in anhydrous toluene (20 ml) was stirred at 110°C for 3 hours. Excess amount of water (50 µl) was added to the mixture and stirred at 60°C for 16 hours. The volatile solvent was distilled off and the crude product was used in the next step without purification. Having the following properties *mlz:* 331 (MH⁺).

### METHOD 29

### (3-{1-:[5-(3-Fluoro-benzyl)-3-methyl-4-oxo-4,5-dihydro-isoxazolo[5,4-d]pyrimidin-6-yl]-2-methyl-propylamino}-propyl)-carbamic acid tert-bu 1 ester

A mixture of 6-(1-amino-2-methyl-propyl)-5-(3-fluoro-benzyl)-3-methyl-5H-isoxazolo[5,4-d]pyrimidin-4-one (method 28) (0.89g, 2.72 mmol) and (3-oxo-propyl)-carbamic acid tert-butyl ester (1.0g, 6.0 mmol) in DCM (20 ml) with dried 4ÅMS was stirred for 1h at room temperature. Then sodium triacetoxyborohydride (0.63g, 3 mmol, 1.2 eq) and 1 drop of acetic acid were added to the mixture. The mixture was stirred at room temperature for 1 day. The mixture was filtered through a 2µ cartridge, the filtrate was concentrated, the crude mixture was purified by ISCO (elute: EtOAc -hexane = 30% - 70%) to give 300 mg, 0.61 mmol (22% yield for 2 steps) of (3-{1-[5-(3-Fluoro-benzyl)-3-methyl-4-oxo-4,5-dihydro-isoxazolo[5,4-d]pyrimidin-6-yl]-2-methyl-propylamino}-propyl)-carbamic acid tert-butyl ester as a white solid. Having the following properties *m*/*z*: 488 (MH⁺), 1H-NMR (300 MHz)) δ : 0.92 (d, 3H), 0.97 (d, 3H), 1.42 (s, 9H), 1.32-1.48 (m, 1H), 1.77-2.01 (m, 3H), 2.36-2.43 (m, 1H), 2.62 (s, 3H), 2.96-3.12 (m, 2H), 3.54 (d, 1H), 2.62 (s, 3H), 2.58-2.70 (m, 1H), 3.65 (d, 2H), 4.89 (d, 1H), 5.22 (d, 1H), 5.88 (d, 1H), 6.52-7.03 (m, 3H), 7.31-7.39 (m, 1H).

### METHOD 30

### {3-[{1-[5-(3-Fluoro-benzyl)-3-methyl-4-oxo-4,5-dihydro-isoxazolo[5,4-d]pyrimidin-6-yl]-2-methyl-propyl}-(4-methyl-benzoyl)-amino]-propyl}-carbamic acid tert-butyl ester

A solution of (3-{1-[5-(3-fluoro-benzyl)-3-methyl-4-oxo-4,5-dihydro-isoxazol[5,4-d]pyrimidin-6-yl]-2-methyl-propylamino}-propyl)-carbamic acid tert-butyl ester (method 29) (300 mg, 0.61 mmol) in DCM (10 ml) was added *p*-toluoyl chloride (1.54g, 1.0 mmol, 1.6 eq) followed by diisopropylethylamine (0.26g. 2.0 mmol). The mixture was stirred at 30-40°C for 1 day. The mixture was then diluted with DCM, washed with saturated sodium bicarbonate aq. The organic phase was dried, filtered, and concentrated. The crude oil was purified by ISCO (solvent: EtOAc -hexane) to give {3-[{1-[5-(3-Fluoro-benzyl)-3-methyl-4-oxo-4,5-dihydro-isoxazolo[5,4-d]pyrimidin-6-yl]-2-methyl-propyl}-(4-methyl-benzoyl)-amino]-propyl}-carbamic acid tert-butyl 1 ester as white solid (204mg, 0.34 mmol) (54% yield). Having the following properties: *m*/*z*: 606 (MH⁺) and 1H-NMR (300 MHz) δ: 0.36 (d, 3H), 0.96 (d, 3H), 1.43 (s, 9H), 1.39-1.48 (m, 1H), 2.39 (s, 3H), 2.66 (s, 3H), 2.56-2.76 (m, 4H), 3.43 (t, 2H), 4.01 (m, 1H), 5.30 (d, 1H), 5.72 (d, 1H), 6.05 (d, 1H), 6.92-7.31 (m, 9H).

### METHOD 31

### 3-Amino-2-thioformyl-but-2-enoic acid ethyl ester

To an ice cold solution of phosphoryl chloride (20 mL, 220 mmol), anhydrous DMF (60 mL) was added dropwise and the resulting solution was added dropwise during 30 min to a stirred solution of the ethyl crotonate (25.83 g, 200 mmol) in anhydrous THF (400 mL) with the temperature maintained at 0 °C. The resulting mixture was allowed to warm to room temperature and stirred overnight and then for 4 h at 30 °C: it was then allowed to stand overnight in a refrigerator. Addition of ether (200 mL) resulted in a yellow oil from which the ether layer was decanted. The resulting oil was washed several times with ether until the ether layer became clear. The oily product was dissolved in DCM (800 mL) and was vigorously shaken with aqueous sodium hydrogen sulfide (2M; 500 mL). The organic layer was separated and the aqueous layer washed with DCM (100 mL). The combined organic layers were washed with water (600 mL), brine (400 mL), dried (Na₂SO₄) and concentrated to get orange crystals. The obtained product was triturated with DCM/hexanes to get pure product as orange crystals (25.6 g, 74%). Having the following properties ¹H NMR (300 MHz) δ: 1.33 (t, 3H), 2.57 (s, 3H), 4.23 (q, 2H), 6.83 (bs, 1H), 10.97 (s, 1H), 13.93 (s, 1H).

### METHOD 32

### 3-Methyl-isothiazole-4-carboxylic acid ethyl ester

To a solution of 3-amino-2-thioformyl-but-2-enoic acid ethyl ester (method 31) (25.6 g, 147 mmol) in ethanol (300 niL), was added m-chloroperbenzoic acid (33.3 g, 77%, 149 mmol) in ethanol (200 mL) dropwise with stirring at room temperature. After the completion of the addition the reaction mixture was heated at 75 °C. for 2 h after which the MS showed the complete disappearance of the starting material. The reaction mixture was diluted with ether (500 mL) and the ethereal solution was washed with 0.1 M NaOH solution (3x500 mL) and once with water (400 mL) dried (Na₂SO₄) and concentrated to get the pure product as light brown oil. Yield 23.5 g (93%). Having the following properties: ¹H NMR (300 MHz) δ : 1.40 (t, 3H), 2.73 (s, 3H), 5.07 (t, 1H), 4.36 (q, 2H), 9.24 (s, 1H).

### METHOD 33

### 3-Methyl-isothiazole-4-carboxylic acid

To a solution of 3-methyl-isothiazole-4-carboxylic acid ethyl ester (method 32) (23.3 g, 136 mmol) in THF (200 mL) aqueous NaOH (6.5 g, 162 mmol, in 100 ml of water) was added and the mixture was stirred at room temperature for 16 h. The TLC of the reaction mixture showed the complete disappearance of the starting material. The reaction mixture was cooled in an ice bath and acidified to pH 5 using 6M HCl and the resultant mixture was extracted with ether (3x100 mL). The ether layers were combined, washed with water (100 mL), brine (100 mL), dried (Na₂SO₄) and concentrated to about 10 mL. Addition of hexanes to the above mixture resulted in the precipitation of the product, which was filtered off, washed with hexanes and dried to provide the pure product as a tan powder. Yield 15.3 g (79%). Having the following properties: ¹H NMR (300 MHz) δ 2.39 (s, 3H), 8.98 (s, 1H).

### METHOD 34

### (3-Methyl-isothiazol-4-yl)-carbamic acid tert-butyl ester

To a solution of 3-methyl-isothiazole-4-carboxylic acid (method 33) (14.8 g, 103 mmol) in anhydrous t-BuOH (100 mL) triethyl amine (10.5 g, 104 mmol) was added followed by the dropwise addition of diphenylphosphoryl azide (28.6 g, 104 mmol) and the resulting mixture was heated at reflux overnight after which the TLC showed the complete disappearance of the starting material. The reaction mixture was cooled to room temperature and poured into ice cold water (500 mL). The aqueous layer was extracted with ether (3x100 mL) and the combined organic layers were washed with satd, NaHCO₃ (100 mL), brine (100 mL) and dried (Na₂SO₄). Concentration of the ether solution provided the crude product, which was purified by column chromatography to get the pure product as light brown crystals. Yield 21.4 g (97%). Having the following properties ¹H NMR (300 MHz) δ 1.53 (s, 9H), 2.40 (s, 3H), 6.50 (s, 1H), 8.66 (s, 1H.).

### METHOD 35

### 4-tert-Butoxycarbonylamino-3-methyl-isothiazole-5-carboxylic acid

To a solution of (3-methyl-isothiazol-4-yl)-carbamic acid tert-butyl ester (method 34) (21.4 g, 100 mmol) in anhydrous THF (200 mL) at -78 °C., LDA (139 mL, 1.8 M solution, 250 mmol) was added dropwise over a period of 1 h. The reaction mixture was stirred at -78 °C for a further 3 h after which powdered dry ice was added and the reaction slowly allowed to warm to room temperature overnight. The reaction mixture was quenched by adding saturated NH₄Cl solution and extracted with ether (3x100 mL) and the combined ether layers were back extracted with satd. NaHCO₃ (3x100 mL). The aqueous layers were combined and acidified to pH 5 using 6M HCl and extracted with ether (4x100 mL). The combined ether layers were dried (Na₂CO₃) and concentrated to give the pure acid as an off white powder. Yield 11 g (39%). Having the following properties: ¹H NMR (300 MHz) δ 1.47 (s, 9H), 2.44 (s, 3H), 8.53 (bs, 1H), 9.68 (bs, 1H).

### METHOD 36

### 4-Amino-3-methyl-isothiazole-5-carboxylic acid

4-tert-Butoxycarbonylamino-3-methyl-isothiazole-5-carboxylic acid (method 35) (11 g, 45 mmol) was dissolved in 50 mL of 4M solution of HCl in 1.4-dioxane (200 mmol) and the resulting solution was stirred at room temperature overnight. The TLC showed the complete disappearance of the starting acid. The reaction was concentrated and the residue was triturated with ether and the precipitated hydrochloride salt was filtered off and washed with ether and dried to provide the product as a light brown powder. Yield 8.2 g (100%). Having the following properties: ¹H NMR (300 MHz, DMSO-d₆) δ 2.30 (s, 3H), 8.85 (bs, 3H).

### METHOD 37

### 3-Methyl-5-propyl-isothiazolo[4,5-d][1,3]oxazin-7-one

To a solution of 4-amino-3-methyl-isothiazole-5-carboxylic acid (method 36) (2.91 g, 15 mmol) in pyridine (20 mL) at 0 °C, was added dropwise a solution of butyryl chloride (3.18 g, 30 mmol) in chloroform (30 mL). The reaction mixture was allowed to warm to room temperature and stirred overnight. Chloroform (200 mL) was added to the reaction mixture followed by 2M HCl (200 mL) and the mixture was stirred. The chloroform layer was further washed with 2M HCl (100 mL), water (100 mL), brine (100 mL) and concentrated. Column purification of the thus obtained crude product provided the pure product as light brown solid. Yield 2 g (64%). Having the following properties: ¹H NMR (300 MHz) δ 1.03 (t, 3H), 1.80- 1.92 (m, 2H), 2.65 (s, 3H), 2.76 (t, 2H).

### METHOD 38

### 6-Benzyl-3-methyl-5-propyl-6H-isothiazolo[4,5-d]pyrimidin-7-one

3-Methyl-5-propyl-isothiazolo[4,5-*d*][1,3]oxazin-7-one (method 37) (200 mg, 1.02 mmol) was taken in a 10 mL, microwavable pyrex tube and benzyl amine (1 g, 9.34 mmol) was added to it. The resulting mixture was heated in a microwave synthesizer (CEM's Discoverer) at 200 °C for 20 min. The MS of the reaction mixture showed the complete disappearance of the starting material and the presence of the product peak at 286 (MH⁺). The reaction mixture was diluted with 1N HCl (10 mL) and extracted with EtOAc (2x30 mL). The combined EtOAc layers were washed with water, brine, dried and concentrated. The thus obtained crude product was purified by column chromatography to isolate the pure product as a white solid. Yield 208 mg (71%). Having the following properties: ¹H NMR (300 MHz) δ 0.98 (t, 3H), 1.76-1.88 (m, 2H), 2.68 (s, 3H), 2.74 (t, 2H), 5.42 (s, 2H), 7.10-7.19 (m, 2H), 7.28-7.39 (m, 3H).

### METHOD 39

### 6-Benzyl-5-(1-bromo-propyl)-3-methyl-6H-isothiazolo[4,5-d]pyrimidin-7-one

To a solution of 6-benzyl-3-methyl-5-propyl-6H-isothiazolo[4,5-d]pyrimidin-7-one (method 38) (208 mg, 0.69 mmol) and sodium acetate (0.5 g, 5 mmol) in acetic acid (10 mL).at 100 °C, a solution of the bromine (0.232 g, 1.46 mmol) in acetic acid (20 mL) was added dropwise over a period of 30 min. The reaction mixture was cooled after the addition and the TLC (eluent 10% EtOAc in hexanes) and MS showed the complete disappearance of the SM and only the product. The reaction mixture was poured into ice water and extracted with EtOAc (3 X 30 mL) and the organic layers were combined and washed with 2% sodium thiosulfate solution (30 mL), water (50 mL), brine (50 mL) and dried (Na₂SO₄). Concentration of the organic layer provided the product and it was pure enough to be used in the next step. Yield 260 mg (99%). Having the following properties: ¹H NMR (300 MHz) δ 0.77 (t, 3H), 2.20-2.54 (m, 2H), 2.70 (s, 3H), 4.67 (t, 1H), 4.95 (d, 1H), 6.25 (d, 1H) 7.10-7.19 (m, 2H), 7.30-7.39 (m, 3H).

### Alternative procedures to prepare certain starting materials

### METHOD 1

### 2-(1-Ethoxy-ethylidene)-malononitrile (alternative procedure)

Triethyl orthoacetate (1.6 L, 9 mol), malononitrile (500 g, 7.57 mol) and glacial acetic acid (25 ml) were placed in a 51 RB flask equipped with a stirrer, thermometer and a Vigreux column (20 x 1 in.) on top of which a distillation condenser was placed. The reaction mixture was heated and ethyl alcohol began to distil when the temperature of the reaction mixture was about 85-90 °C. After about 3h., the temperature of the reaction mixture reached 140°C. Then the reaction was concentrated in a rotary evaporator to remove the low-boiling materials and the residue was stirred with isopropyl alcohol (11) and cooled in an ice bath. The crystallized product was filtered off washed with isopropyl alcohol (200 ml), hexanes (600 ml) and dried at 50°C. in a vacuum oven overnight to yield 2-(1-ethoxy-ethylidene)-malononitrile (974 g, 94%) as a golden yellow solid [mp 92. °C (lit.90-92 °C, MCCall. M. A. J. Org. Chem. 1962, 27, 2433-2439.)].

### METHOD 2

### (2E)-2-Cyano-3-ethoxybut-2-enethioamide (alternative procedure)

2-(1-Ethoxy-ethylidene)-malononitrile (method 1) (300 g, 2.2 mol) was dissolved in anhydrous benzene (3.11, slight warming required) and 20 ml of triethylamine was added. The mixture was mechanically stirred and hydrogen sulfide was bubbled into this solution for 2 h and a solid formed. Then N₂ was bubbled through the reaction mixture for 40 min. The precipitated solid was filtered off, washed with cold benzene (200 ml) and dried in a vacuum oven overnight to isolate (2*E*)-2-cyano-3-ethoxybut-2-enethioamide (332 g, 88%) as light brown crystals.

### METHOD 3

### (2E)-3-Amino-2-cyanobut-2-enethioamide (alternative procedure)

(2E)-2-Cyano-3-ethoxybut-2-enethioamide (method 2) (150 g, 0.88 mol) was dissolved in 7M solution of ammonia in methanol (2.9 L) and stirred at r.t. overnight. The reaction mixture was concentrated and the residue was crystallized from hot water (1. L) to provide (2E)-3-amino-2-cyanobut-2-enethioamide (111.6 g, 89%) as brown crystals. ¹H NMR (300 MHz, DMSO-d₆) δ 2.22 (s, 3H), 7.73 (bs, 1H), 8.53 (bs, 1H), 9.01 (bs, 1H), 11.60 (bs, 1H).

### METHOD 4

### 5-Amino-3-methylisothiazole-4-carbonitrile (alternative procedure)

To a stirred solution of (2E)-3-amino-2-cyanobut-2-enethioamide (method 3) (111 g, 0.78 mol) in methanol (2 L) was added dropwise 200 ml of 35% hydrogen peroxide over a period of 30 min. After the completion of the addition the mixture was stirred at 60°C for 3h after which the TLC showed the completion of the reaction. The reaction mixture was evaporated to 300 ml in a rotary evaporator and cooled in an ice-bath. The crystallized product was filtered off and washed with isopropyl alcohol (100 ml) and dried in vacuum at 50 °C overnight to provide 5-amino-3-methylisothiazole-4-carbonitrile (105.63 g, 96%) as a light yellow crystalline solid. ¹H NMR (300 MHz, DMSO-d₆) δ 2.24 (s, 3H), 8.00 (bs, 2H).

### METHOD 12

### N-(4-Cyano-3-methyl-isothiazol-5-yl)-3-methyl-butyramide (alternative procedure)

To a solution of 5-amino-3-methylisothiazole-4-carbonitrile (method 4) (105.6 g, 0.76 mol) in pyridine (250 ml) at 0 °C, isovaleryl chloride (100 g, 0.83 mol) in chloroform (300 ml) was added dropwise. After the completion of the addition the reaction mixture was allowed to warm to r.t. and stirred overnight. The TLC and the MS showed the complete disappearance of the starting material and the reaction mixture was diluted with CHCl₃ (600 ml), washed with water (200 ml), 2N HCl (600 ml), satd. NaHCO₃ (200 ml), brine (200 ml) and dried over Na₂SO₄. Concentration of the CHCl₃ layer provided the crude product which was triturated from DCM/hexanes (1/10) and filtered off to isolate N-(4-cyano-3-methyl-isothiazol-5-yl)-3-methylbutyramide (149.7 g, 88%) as an off-white crystalline solid. ¹H NMR (300 MHz) δ 1.04 (d, 6H), 2.18-2.32 (m, 1H), 2.46 (d, 2H), 2.53 (s, 3H), 9.87 (bs, 1H).

### METHOD 13

### 3-Methyl-5-(3-methyl-butyrylamino)-isothiazole-4-carboxylic acid amide (alternative procedure)

To a solution of N-(4-cyano-3-methyl-isothiazol-5-yl)-3-methyl-butyramide (method 12) (72 g, 322 mmol) in 30% aqueous NH₄OH (2.1 L), was added dropwise 1.3 L of hydrogen peroxide at 40°C. After 20 min the temperature of the reaction mixture rose to 60°C. The addition was completed in 1.5 h. After an additional 2 h the MS showed the completion of the reaction. The reaction mixture was cooled in ice and con HCl was slowly added with cooling till the pH of the reaction mixture turns 7.6. The precipitated product was filtered and dried in vacuum oven to get the pore amide (36 g, 46%). The filtrate was saturated with NaCl and extracted with super solvent (34:66, t-butanol:1,2-dichloroethane) and the combined organic extracts were washed with water (500 ml), brine (600 ml) and dried (Na₂SO₄) and concentrated. The residue on trituration with EtOAc/hexanes (1/4) provided an additional 9.8 g of pure product. Total yield of 45.8 g (58%) 3-methyl-5-(3-methyl-butyrylamino)-isothiazole-4-carboxylic acid amide. ¹H NMR (300 MHz) δ 1.03 (d, 6H), 2.24 (m, 1H), 2.43 (d, 2H), 2.69 (s, 3H), 5.98 (bs, 2H), 11.77 (bs, 1H).

### METHOD 14

### 6-Isobutyl-3-methyl-5H-isothiazolo[5.4-d]pyrimidin-4-one (alternative procedure)

The 3-methyl-5-(3-methyl-butyrylamino)-isothiazole-4-carboxylic acid amide (method 13) (45.8 g, 190 mmol) was suspended in 700 ml of 30% NH₃ and then was heated to 140°C for 5h in a pressure reactor. The mixture was poured into a 4 L beaker and cooled in an ice bath. To the cold solution con HCl (560 ml) was added dropwise to pH 7.5 and a white precipitate was formed. The precipitated product was filtered off, washed with water (100 ml) and dried under vacuum overnight. 6-Isobutyl-3-methyl-5H-isothiazolo[5,4-d]pyrimidin-4-one (11 g, 26%) was isolated as an off-white powder. ¹H NMR (300 MHz) δ 1.05 (d, 6H), 2.32 (m, 1H), 2.69 (d, 2H), 2.82 (s, 3H).

### METHOD 15

### 5-Benzyl-6-isobutyl-3-methyl-5H-isothiazolo[5,4-d]pyrimidin-4-one (alternative procedure)

To a solution of 6-isobutyl-3-methyl-5H-isothiazolo[5,4-d]pyrimidin-4-one (method 14) (11 g, 49 mmol) in 60 ml of anhydrous DMF at 0 °C, was added 13.8 g (100 mmol) of anhydrous K₂CO₃ followed by benzyl bromide (9.3 g, 54 mmol) and the mixture was stirred at 0-20 °C overnight. The TLC of the reaction mixture showed the complete disappearance of the SM. The reaction mixture was poured into ice-cold water and extracted with EtOAc (3X100 ml). The combined extracts were washed with water (100 ml), brine (100 ml), dried (Na₂SO₄) and concentrated. The TLC and the ¹H NMR showed the presence of two products N alkylated as well as *O*-alkylated products in a ratio of 75:25. The products were separated by column (silica gel) chromatography using 10% EtOAc in hexanes. The major N-alkylated product 5-benzyl-6-isobutyl-3-methyl-5H-isothiazolo[5,4-d]pyrimidin-4-one was isolated as white crystalline solid (10.8 g, 70%). ¹H NMR (300 MHz) δ 0.94 (d, 6H), 2.23-2.37 (m, 1H), 2.64 (d, 2H), 2.82 (s, 3H), 5.38 (s, 2H), 7.10-7.38 (m, 5H).

### METHOD 16

### 5-Benzyl-6-(1-bromo-2-methyl-propyl)-3-methyl-5H-isothiazolo[5,4-d]pyrimidin-4-one (alternative procedure)

To a solution of 5-benzyl-6-isobutyl-3-methyl-5H-isothiazolo[5,4-d]pyrimidin-4-one (method 15) (5.81 g, 18.5 mmol) and sodium acetate (10 g) in acetic acid (100 ml) at 100 °C, a solution of the bromine (6 g, 38 mmol) in acetic acid (60 ml) was added dropwise over a period of 20 minutes. The reaction mixture was stirred at that temperature for 30 min and cooled and the TLC (eluent 10% EtOAc in hexanes) and MS showed the complete disappearance of the SM and only the product. The reaction mixture was poured into ice water and extracted with EtOAc (3 X 60 ml) and the organic layers were combined and washed with 2% sodium thiosulfate solution (60 ml), water (100 ml), brine (100 ml) and dried over Na₂SO₄. Concentration of the organic layer provided 5-benzyl-6-(1-bromo-2-methyl-propyl)-3-methyl-5H-isothiazolo[5,4-d]pyrimidin-4-one (7.27 g, 99%) as white crystalline solid. ¹H NMR (300 MHz) δ 0.54 (d, 3H), 1.11 (d, 3H), 2.62-2.76 (m, 1H), 2.83 (s, 3H), 4.42 (d, 1H), 4.80 (d, 1H), 6.22 (d, 1H), 7.12-7.42 (m, 5H).

### METHOD 17

### 6-(1-Azido-2-methyl-propyl)-5-benzyl-3-methyl-5H-isothiazolo[5,4-d]pyrimidin-4-one (alternative procedure)

To a solution of 5-benzyl-6-(1-bromo-2-methyl-propyl)-3-methyl-5H-isothiazolo[5,4-d]pyrimidin-4-one (method 16) (7.27 g, 18.5 mmol) in anhydrous DMF (60 ml), sodium azide (2.33 g, 37 mmol) was added and the mixture was stirred at room temperature for 2 hour. The TLC of the RM showed the complete disappearance of the starting bromide. The reaction mixture was poured into ice water (300 ml) and extracted with EtOAc (3 X 100 ml). The organic layer was washed with water (100 ml), brine (100 ml) and dried (Na₂SO₄). Concentration of the organic layer provided the crude product which was purified by column (silica gel) chromatography using 30% EtOAc in hexanes as eluent to isolate 6-(1-azido-2-methyl-propyl)-5-benzyl-3-methyl-5H-isothiazolo[5,4-d]pyrimidin-4-one (6.16 g, 94%) as a low melting solid. ¹H NMR (300 MHz) δ 0.57 (d, 3H), 1.07 (d, 3H), 2.50-2.74 (m, 1H), 2.98 (s, 3H), 3.71 (d, 1H), 5.05 (d, 1H), 5.78 (d, 1H), 7.12-7.40 (m, 5H).

### METHOD 18

### 6-(1-Amino-2-methyl-propyl)-5-benzyl-3-methyl-5H-isothiazolo[5,4-d]pyrimidin-4-one (alternative procedure)

To a solution of 6-(1-azido-2-methyl-propyl)-5-benzyl-3-methyl-5H-isothiazolo[5,4-d]pyrimidin-4-one (method 17) (6.8 g, 19.2 mmol) in methanol (400 ml) was added 5% Pd/C (1 g, 20% by wt.) and the resulting mixture was stirred at r.t. in an atmosphere of H₂ and the progress of the reaction was monitored by MS. After the disappearance of the starting material the reaction mixture was filtered through celite and washed with EtOAc. Concentration of the filtrate provided 6-(1-amino-2-methyl-propyl)-5-benzyl-3-methyl-5H-isothiazolo[5,4-d]pyrimidin-4-one (5.42 g, 86%).

### Method 40

### 5-Amino-3-methylisothiazole-4-carboxamide

To a chilled solution of sulfuric acid (7.2 volumes, 12.9 equivs) was charged 5-amino-3-methylisothiazole-4-carbonitrile (method 4) (1.0 equiv). The temperature was maintained below 55°C. The reaction mixture was heated to 70°C and held for 1 hour until TLC showed disappearance of starting material. The mixture was cooled to 60-65°C before the ammonia (21 volumes) was charged to pH 10. The mixture was cooled to 20°C, aged overnight and filtered. The resulting solid was washed with dilute ammonia (3.6 volumes) and dried at 40°C to give a pale brown solid (typical yield 80%). ¹H NMR (300MHz, DMSO-d₆) δ 2.46(s, 3H), 6.28 (s, 1H).

### METHOD 14

### 6-Isobutyl-3-methyl-5H-isothiazolo[5,4-d]pyrimidin-4-one (alternative procedure)

To a 2 L flask equipped with Dean Stark was charged 5-amino-3-methylisothiazole-4-carboxamide (method 41) (1 equiv), p-toluene sulphonic acid (0.049 equiv), DMF.(9.75 volumes). The reaction was stirred until a solution was obtained and isovaleraldehyde (1.10 equiv) and toluene (4.9 volumes) were added. The resulting mixture was heated to 130°C and held at reflux for I hour removing water via a Dean Stark apparatus. Once the reaction was complete toluene was removed under vacuum distillation. Sodium bisulfite (2.50 equiv) was charged and the mixture was held at 115°C for 7 hours, then cooled to room temperature overnight. The solid was removed by filtration through harborlite and washed with DMF (1 volume). Analysis showed conversion to product and the reaction was heated to 50°C. water (15 volumes) was added and the resulting precipitate was cooled to room temperature and held for 1 h. The product was isolated by filtration and washed with water (2x0.5 volumes), dried to give a pale brown solid (typical yield 89%).

### EXAMPLE A

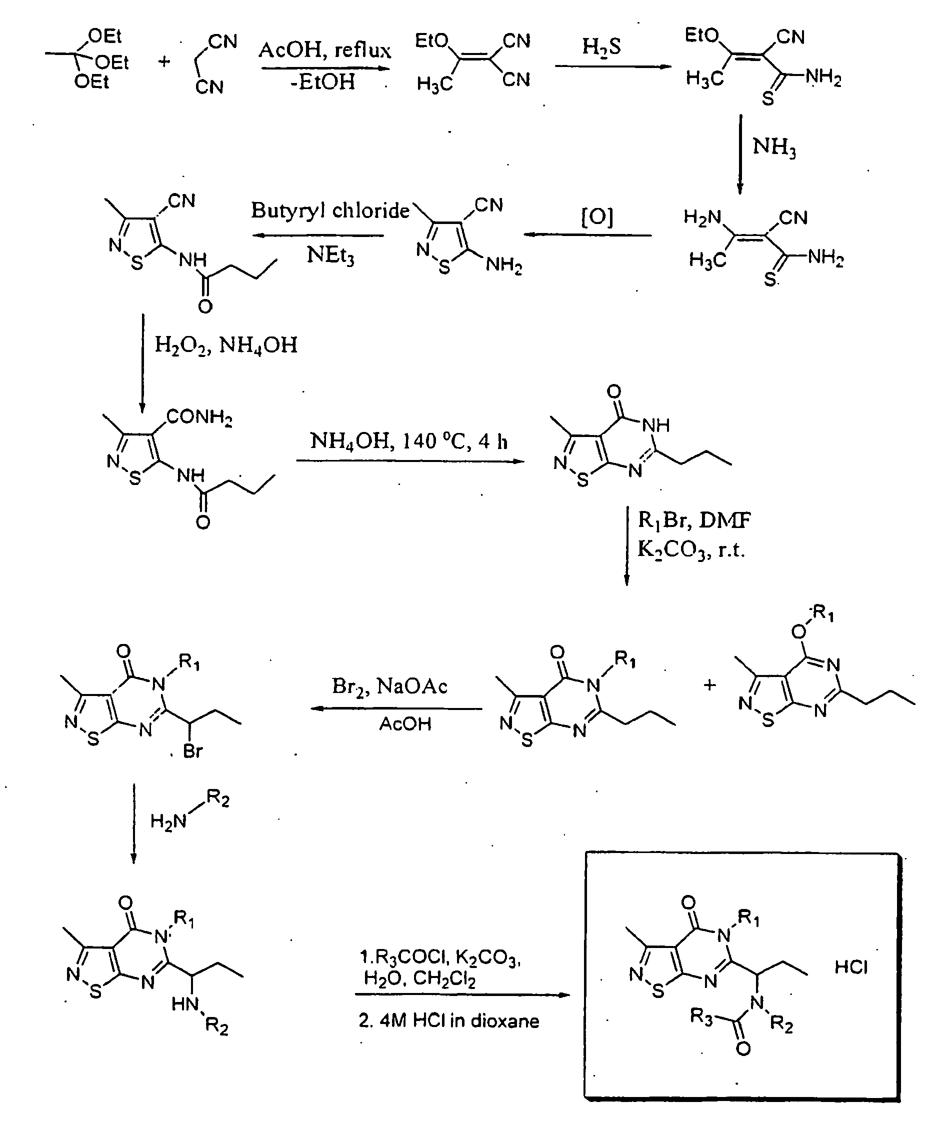

### EXAMPLE A1

The following compound was synthesized according to synthetic scheme A above:

### Example A1

### N-(3-Amino-propyl)-N-{1-[5-(3-fluoro-benzyl)-3-methyl-4-oxo-4,5-dihydro-isothiazolo[5,4-d]pyrimidin-6-yl]-propyl-4-methyl-benzamide hydrogen chloride

{3-[{1-[5-(3-Fluoro-benzyl)-3-methyl-4-oxo-4,5-dihydro-isothiazolo[5,4-d]pyrimidin-6-yl]-propyl}-(4-methyl-benzoyl)-amino]-propyl}-carbamic acid tert-butyl ester (method 11) (0.030 g, 0.049 mmol) was dissolved in 4M HCl in 1,4-dioxane and the mixture was stirred at r.t. for 30 min and the LC/MS showed the complete disappearance of the starting material. The reaction mixture was concentrated in a rotary evaporator and the residue was triturated with ether. The precipitated product was filtered off and dried *in vacuo* to yield N-(3-amino-propyl)-N-{1-[5-(3-fluoro-benzyl)-3-methyl-4-oxo-4,5-dihydro-isothiazolo[5,4-d]pyrimidin-6-yl]-propyl}-4-methyl-benzamide hydrogen chloride (0.0215 g, 80%), *m*/*z* 508 (MH⁺), ¹H NMR (DMSO-d₆, 90 °C) δ ppm 0.41-.0.46 (t, 3H), 1.13- 1.28 (m, 1H), 1.38 - 1.53 (m, 1H), 1.61 - 1.78 (m, 1H), 1.88 - 2.01 (m, 1H), 2.11 (s, 3H), 2.14 - 2.23 (m, 2H),) 12.50 (s, 3H), 3.08 - 3.18 (m, 2H), 4.63 (br, 1H), 5.22 (br, 1H), 5.45-5.55 (d, 1H), 6.60 - 7.16 (m, 8H), 7.43-7.63 (br s, 3H).

| **Ex.** | **Compound** | **¹H NMR** | **m/z** | **SM** |
|---|---|---|---|---|
| **A1** | N-(3-Amino-propyl)-N-{1-[5-(3-fluoro-benzyl)-3-methyl-4-oxo-4,5-dihydro-isothiazolo[5,4-d]pyrimidin-6-yl]-propyl}-4-methyl-benzamide hydrogen chloride | (DMSO-d₆, 90°C) δ ppm 0.41-0.46 (t, 3H), 1.13- 1.28 (m, 1H), 1.38 - 1.53 (m, 1H), 1.61 - 1.78 (m, 1H), 1.88-2.01 (m,1H), 2.11 (s, 3H), 2.14 - 2.23 (m, 2H), ) 2.50 (s, 3H), 3.08 - 3.18 (m, 2H), 4.63 (br, 1H), 5.22 (br, 1H), 5.45-5.55 (d, 1H), 6.60 - 7.16 (m, 8H), 7.43-7.63 (br s, 3H) | m/z 508 (MH⁺) | Method 11 |

### EXAMPLE B

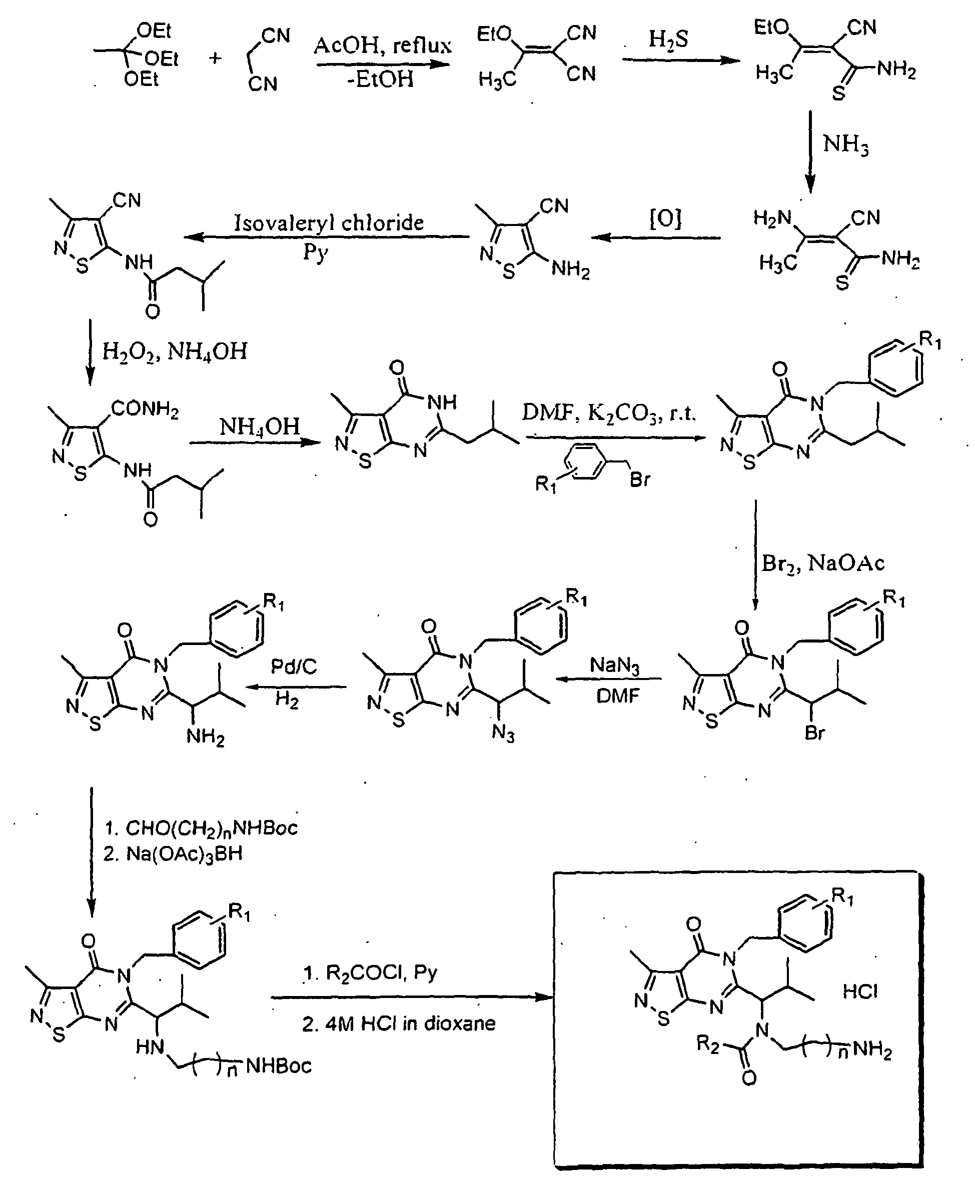

### EXAMPLES B1-B6

The following compounds were synthesized according to synthetic scheme B above:

### Example B1

### N-(2-Amino-ethyl)-N-[1-(5-benzyl-3-methyl-4-oxo-4,5-dihydro-isothiazolo[5,4-d]pyrimidin-6-yl)-2-methyl-propyl]-4-bromo-benzamide hydrogen chloride

{2-[[1-(5-Benzyl-3-methyl-4-oxo-4,5-dihydro-isothiazolo[5,4-d]pyrimidin-6-yl)-2-methyl-propyl]-(4-bromo-benzoyl)-amino]-ethyl}-carbamic acid tert-butyl ester (method 21b) (0.040 g, 0.061 mmol) was dissolved in 4M HCl in 1,4-dioxane and the mixture was stirred at r.t. for 30 min and the LC/MS showed the complete disappearance of the starting material. The reaction mixture was concentrated in a rotary evaporator and the residue was triturated with ether. The precipitated product was filtered off and dried *in vacuo* to yield N-(2-amino-ethyl)-N-[1-(5-benzyl-3-methyl-4-oxo-4,5-dihydro-isothiazolo[5,4-d]pyrimidin-6-yl)-2-methyl-propyl]-4-bromo-benzamide hydrogen chloride (0.0345 g, 96%), m/z 554, 556 (MH⁺), ¹H NMR (DMSO-d₆, 90 °C) δ: 0.37-0.0.38 (d, 3H), 0.89-0.91 (d, 3H), 2.28-2.38 (m, 1H), 2.58-2.69 (m, 2H), 2.77 (s, 3H), 3.61-3.71 (m, 2H), 4.99 (br, 1H), 5.54 (br d, 1H), 5.89-5.93 (d, 1H), 7.20-7.70 (m, 9H), 7.84 (br, 3H).

The following compounds were prepared by the procedure of Example B1.

| **Ex.** | **Compound** | **¹H NMR** | **m/z** | **SM** |
|---|---|---|---|---|
| **B1** | N-(2-Amino-ethyl)-N-[1-(5-benzyl-3-methyl-4-oxo-4,5-dihydro-isothiazolo[5,4-d]pyrimidin-6-yl)-2-methyl-propyl]-4-bromo-benzamide hydrogen chloride | (DMSO-d₆, 90 °C) δ: 0.37-0.0.38 (d, 3H), 0.89-0.91 (d, 3H), 2.28-2.38 (m, 1H), 2.58-2.69 (m, 2H), 2.77 (s, 3H), 3.61-3.71 (m, 2H), 4.99 (br, 1H), 5.54 (br d, 1H), 5.89-5.93 (d, 1H), 7.20-7.70 (m, 9H), 7.84 (br, 3H) | m/z 554, 556 (MH⁺) | Method 21b |
| **B2** | N-(2-Amino-ethyl)-N-[1-(5-benzyl-3-methyl-4-oxo-4,5-dihydro-isothiazolo[5,4-d]pyrimidin-6-yl)-2-methyl-propyl]-3-fluoro-4-methyl-benzamide hydrogen chloride | (DMSO-d₆, 90°C) δ: 0.14-0.16 (d, 3H), 0.66-0.68 (d, 3H), 2.10-2.20 (m and s, 4H), 2.40-2.50 (m, 2H), 2.55 (s, 3H), 3.40-3.50 (m, 2H), 4.78 (b, 1H), 5.30 (b, 1H), 5.60-5.70 (d, 1H), 6.90-7.23 (m, 8H), 7.50-7.70 (bs, 3H) | m/z 508 (MH⁺) | Method 21c |
| **B3** | N-(3-Amino-propyl)-N-[1-(5-benzyl-3-methyl-4-oxo-4,5-dihydro-isothiazolo[5,4-d]pyrimidin-6-yl)-2-methyl-propyl]-3-fluoro-4-methyl-benzamide hydrogen chloride | (DMSO-d₆, 90 °C) δ: 0.46-0.48 (d, 3H), 0.90-0.92 (d, 3H), 1.2-1.48 (m, 1H), 1.51-1.72 (m, 1H), 2.29-2.40 (m, 5H), 2.70-2.77 (m,s, 4H), 3.35-3.40 (t, 2H), 5.00-5.10 (d, 1H), 5.60-5.65 (d, 1H), 5.90-5.94 (d, 1H), 7.07-7.38 (m, 8H), 7.71 (b, 2H) | m/z 522 (MH⁺) | Method 21 d |
| **B4** | N-(3-Amino-propyl)-N-[1-(5-benzyl-3-methyl-4-oxo-4,5-dihydro-isothiazolo[5,4-d]pyrimidin-6-yl)-2-methyl-propyl]-4-bromo-benzamide hydrogen chloride | (DMSO-d₆, 90 °C) δ: 0.46-0.47 (d, 3H), 0.90-0.92 (m, 3H), 1.15-1.25 (m, 1H), 1.45-1.60 (m, 1H), 2.29-2.33 (t, 2H), 2.65-2.77 (m, s, 4H), 3.34-3.38 (m, 2H), 5.05-5.10 (d, 1H), 5.60-5.66 (m, 1H), 5.90-5.94 (d, 1H), 7.27-7.38 (m, 7H), 7.55-7.66 (br m, 4H) | m/z 568, 570 (MH⁺) | Method 21e |

The following compounds may be prepared by the procedure of Example B1.

| **Ex.** | **Compound** | **SM** |
|---|---|---|
| **B5** | N-(3-Amino-propyl)-N-{ 1-[5-(3-fluoro-benzyl)-3-methyl-4-oxo-4,5-dihydro-isothiazolo[5,4-d]pyrimidin-6-yl]-2-methyl-propyl} -4-methyl-benzamide hydrogen chloride | Method 21a |
| **B6** | N-(2-Amino-ethyl)-N-[1-(5-benzyl-3-methyl-4-oxo-4,5-dihydro-isothiazolo[5,4-d]pyrimidin-6-yl)-2-methyl-propyl]-4-methyl-benzamide hydrogen chloride | Method 21 |

### EXAMPLE C

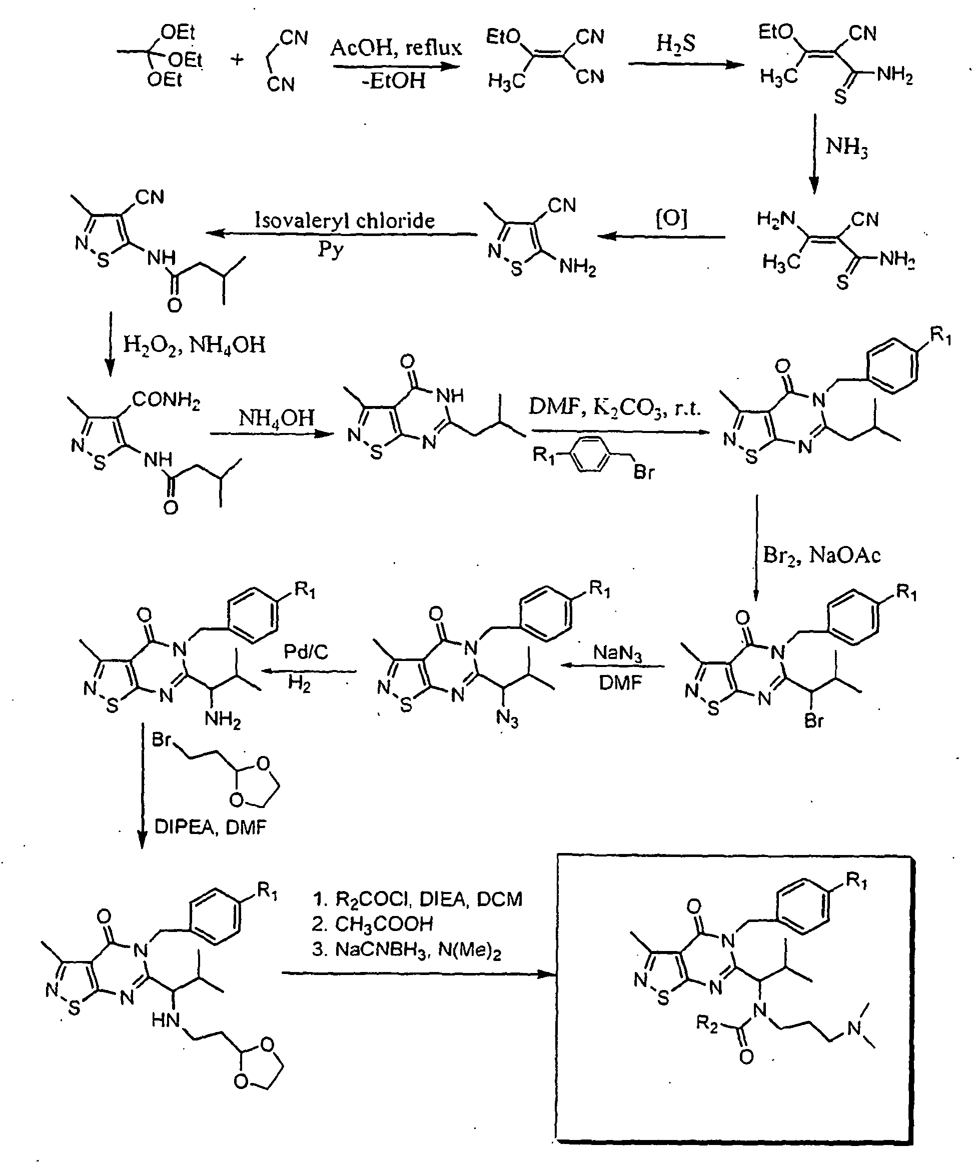

### EXAMPLES C1-C3

The following compounds were synthesized according to synthetic scheme C above:

### Example C2

### N-[1-(5-Benzyl-3-methyl-4-oxo-4,5-dihydro-isothiazolo[5,4-d]pyrimidin-6-yl)-2-methyl-propyl-4-bromo-N-(3-dimethylamino-propyl)-benzamide

To a solution of N-[1-(5-benzyl-3-methyl-4-oxo-4,5-dihydro-isothiazolo[5,4-d]pyrimidin-6-yl)-2-methyl-propyl]-4-bromo-N-(3-oxo-propyl)-benzamide (method 22) (1 g, 1.76 mmol) in methanol (20 mL) two drops of acetic acid were added followed by the addition of dimethylamine (1mL, 2M solution in THF) and sodium cyanoborohydride (0.314 g, 5 mmol) and the mixture was stirred at room temperature for 3 h. The reaction mixture was concentrated and the residue was dissolved in DCM (100 mL) and the organic layer was washed with satd. NaHCO₃ (3x100 mL). The organic layer was concentrated and the crude product was purified by column chromatography using 0-10% MeOH in EtOAc. The pure product fractions were concentrated and the thus obtained foam was crystallized from ether/hexanes to get the product as white crystalline solid. Yield = 0.366 g (35%). Having the following properties m/z 596, 598 (MH⁺); ¹H-NMR (300 MHz, 25°C) δ 0.31-0.36 (d, 3H), 0.67-0.77 (m, 1H), 0.89-0.94 (d, 3H), 1.19-1.27 (m, 1H), 1.65-1.83 (m, s, 8H), 2.66-2.76 (m, 1H), 2.89 (s, 3H), 3.30-3.40 (m, 2H), 5.17-5.23 (d, 1H), 5.71-5.75 (d, 1H), 6.12-6.17 (d, 1H), 7.25-7.41 (d, m, 7H), 7.55-7.58 (d, 2H).

The following compounds were synthesised according to Example C2 above.

| **Ex.** | **Compound** | **¹H NMR** | **m/z** | **SM** |
|---|---|---|---|---|
| **C1** | N-[1-(5-Benzyl-3-methyl-4-oxo-4,5-dihydro-isothiazolo[5,4-d]pyrimidin-6-yl)-2-methyl-propyl]-N-(3-dimethylamino-propyl)-4-methyl-benzamide | (300 MHz) δ: 0.34-0.36 (d, 3H), 0.68-0.75 (m, 1H), 0.93-0.96 (d, 3H), 1.22-1.30 (m, 1H), 1.65-1.87 (br m, s, s, 8H), 2.37 (s, 3H), 2.66-2.72 (m, 1H), 2.87 (s, 3H), 3.35-3.41 (m, 2H), 5.22-5.27 (d, 1H), 5.73-5.76 (d, 1H), 6.12-6.17 (d, 1H), 7.22-7.41 (m, 9H) | m/z 532 (MH⁺) | Method 22a |
| **C2** | N-[1-(5-Benzyl-3-methyl-4-oxo-4,5-dihydro-isothiazolo[5,4-d]pyrimidin-6-yl)-2-methyl-propyl]-N-(3-dimethylamino-propyl)-4-bromo-benzamide | (300 MHz, 25°C) δ 0.31-0.36 (d, 3H), 0.67-0.77 (m. 1H), 0.89-0.94 (d, 3H), 1.19-1.27 (m, 1H), 1.65-1.83 (m, s, SH), 2.66-2.76 (m, 1H), 2.89 (s, 3H), 3.30-3.40 (m, 2H), 5.17-5.23 (d, 1H), 5.71-5.75 (d, 1H), 6.12-6.17 (d, 1H), 7.28-7.41 (d, m, 7H), 7.55-7.58 (d, 2H) | m/z 597 (MH⁺) | Method 22 |
| **C3** | N-[1-(5-Benzyl-3-methyl-4-oxo-4,5-dihydro-isothiazolo[5,4-d]pyrimidin-6-yl)-2-methyl-propyl]-N-(3-dimethylamino-propyl)-3-fluoro-4-methyl-benzamide | (300 MHz, 25 °C) δ: 0.35-0.40 (d, 3H), 0.68-0.78 (m, 1H), 0.92-0.94 (d, 3H), 1.20-1.30 (m, 1H), 1.65-1.83 (br m, s, s, 8H), 2.30 (s, 3H), 2.67-2.75 (m, 1H), 2.87 (s, 3H), 3.35-3.44 (t, 2H), 5.17-5.23 (d, 1H), 5.71-5.74 (d, 1H), 6.11-6.16 (d, 1H), 6.99-7.39 (m, 8H) | m/z 540 (MH⁺) | Method 22b |

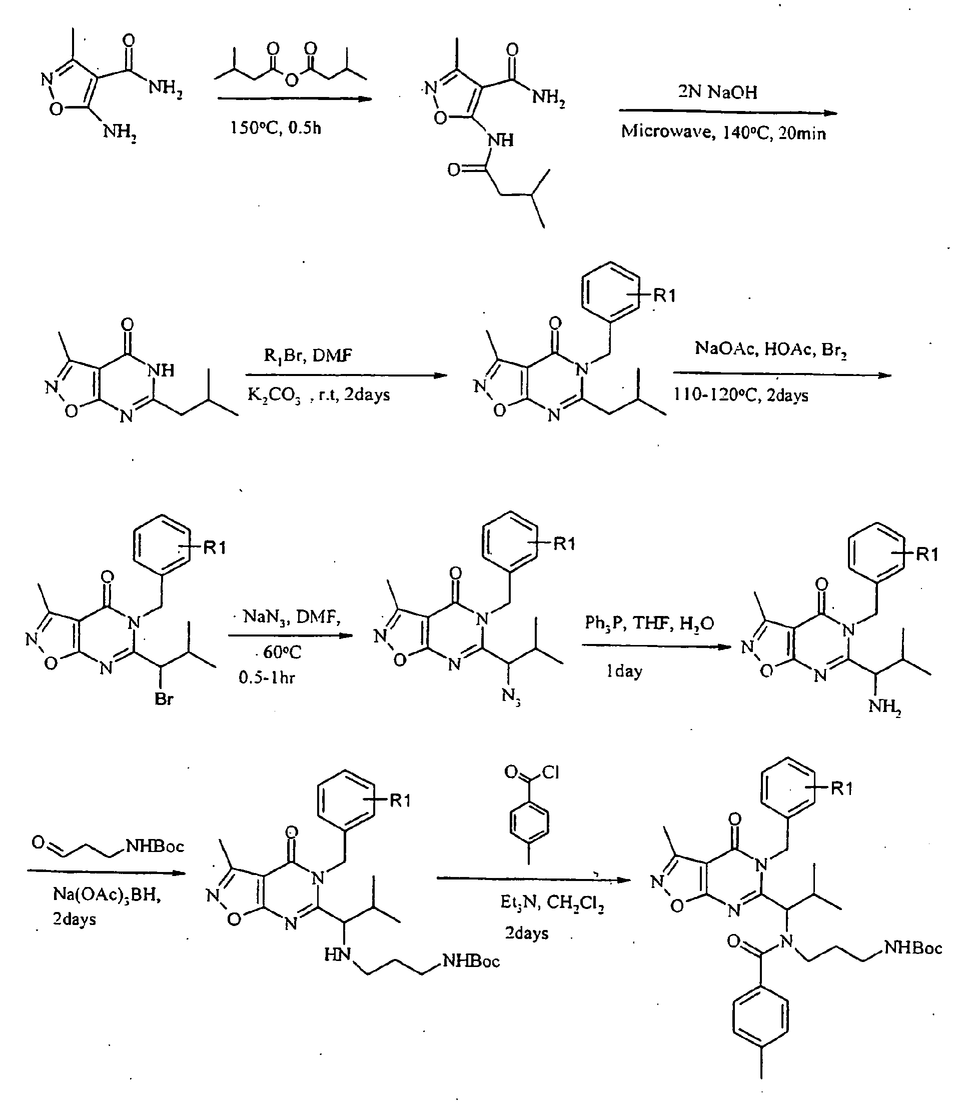

### EXAMPLE D

The following compound may be synthesized according to synthetic scheme D above:

### EXAMPLE D1

### N-(3-Amino-propyl)-N-{1-[5-(3-fluoro-benzyl)-3-methyl-4-oxo-4,5-dihydro-isoxazolo[5,4-d]pyrimidin-6-yl)-2-methyl-propyl}-4-methyl-benzamide hydrogen chloride

A solution of {3-[{1-[5-(3-fluoro-benzyl)-3-methyl-4-oxo-4,5-dihydro-isoxazolo[5,4-d]pyrimidin-6-yl]-2-methyl-propyl}-(4-methyl-benzoyl)-amino]-propyl}-carbamic acid tert-butyl ester (method 30) (100mg, 0.165 mmol) in 5 ml of 4 M HCl in dioxane could be stirred at room temperature for 2hr. The solvent could be distilled off by vacuo and the residue dried at 40-50°C overnight under vacuum to give N-(3-amino-propyl)-N-{1-[5-(3-fluoro-benzyl)-3-methyl-4-oxo-4,5-dihydro-isoxazolo[5,4-d]pyrimidin-6-yl]-2-methyl-propyl} -4-methyl-benzamide as the HCl salt.

| **Ex.** | **Compound** | **SM** |
|---|---|---|
| **D1** | N-(3-Amino-propyl)-N-{1-[5-(3-fluoro-benzyl)-3-methyl-4-oxo-4,5-dihydro-isoxazolo [5,4-d]pyrimidin-6-yl]-2-methyl-propyl}-4-methyl-benzamide hydrogen chloride | Method 30 |

### EXAMPLE E

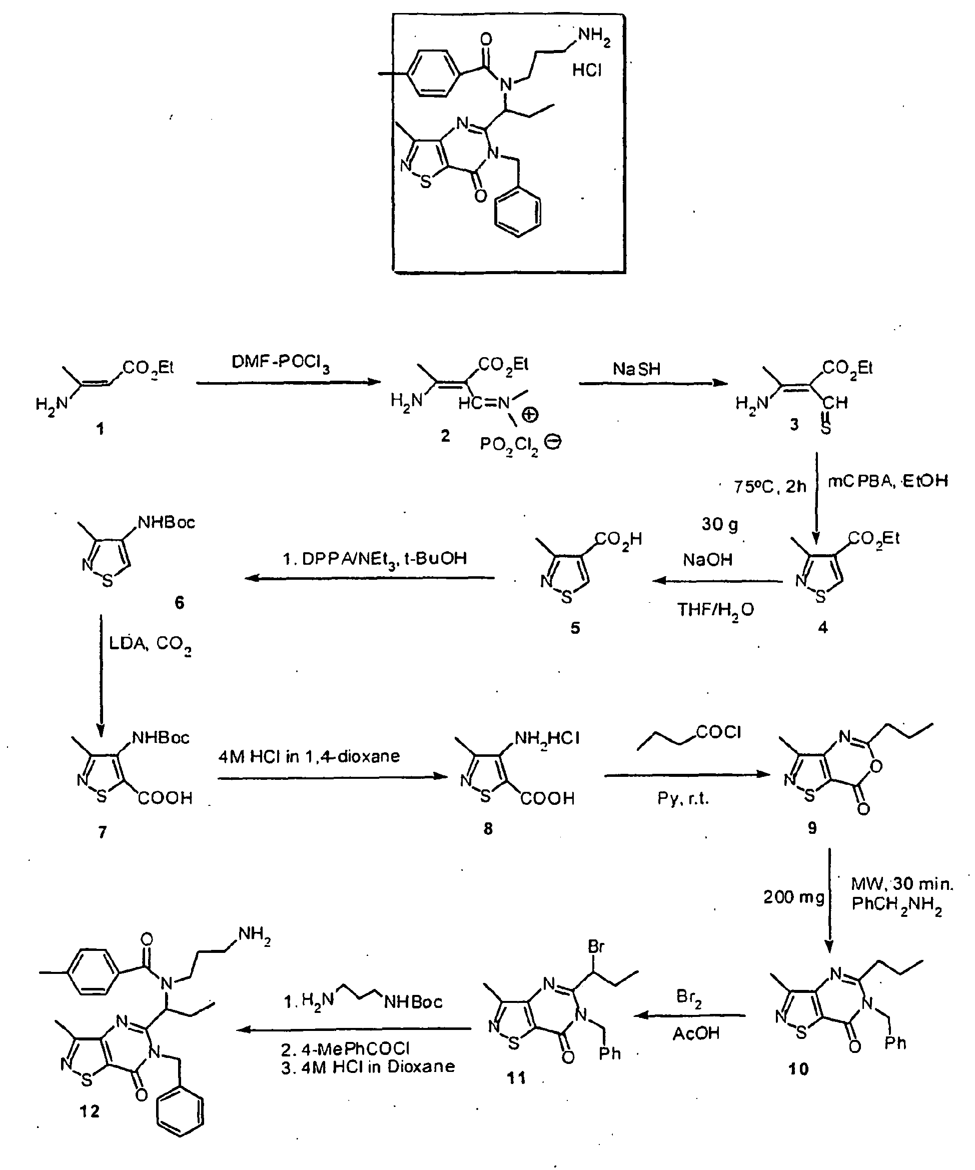

### EXAMPLE E

The following compound was synthesized according to synthetic scheme E above:

### EXAMPLE E1

### N-(3-Amino-propyl)-N-[1 -(6-benzyl-3-methyl-7-oxo-6,7-dihydro-isothiazolo[4,5-d]pyrimidin-5-yl)-propyl]-4-methyl-benzamide hydrogen chloride

To a solution of 6-benzyl-5-(1-bromo-propyl)-3-methyl-6*H*-isothiazolo[4,5-*d*]pyrimidin-7-one (method 39) (260 mg, 0.70 mmol) in anhydrous DMF (10 mL), ethyl diisopropylamine (387 mg, 3 mmol) and *N*-(3-aminopropyl)carbamic acid *tert*-butyl ester (174 mg, 1 mmol) were added at room temperature and the mixture was stirred at room temperature for 1 h after which the MS analysis showed the complete disappearance of the starting bromide and only the product peak at 472 (MH⁺) was observed. The reaction mixture was diluted with water (100 mL) and extracted with EtOAc (3x60 mL). The combined organic extracts were dried and concentrated to get the crude amine, which was dissolved, in chloroform (40 mL) and diisopropylethylamine (387 mg, 3 mmol) was added and the mixture was heated to 60 °C. To the stirred hot solution *p-*toluoyl chloride (154 mg, 1 mmol) in chloroform (20 mL) was added dropwise and the mixture was refluxed for 12 h after which the MS showed the complete disappearance of the amine and only the product peak at 590 (MH⁺). The reaction mixture was concentrated and the crude product was purified by column chromatography to isolate the pure acylated product (80 mg, 20% overall from bromide), which was treated with 4M HCl in 1,4-dioxane (10 mL) for 30 min. The dioxane was evaporated in a rotary evaporator and the residue was dissolved in water and freeze dried to get the pure product as a white fluffy solid. Yield 60 mg (16% overall from bromide). Having the following properties: *m*/*z* 490 (MH⁺); ¹H NMR (300 MHz, DMSO-d₆, 96 °C) δ 0.65 (t, 3H), 1.36-1.50 (m, 1H), 1.60-1.72 (m, 1H), 1.88-1.99 (m, 1H), 2.14-2.26 (m, 1H), 2.35 (s, 3H), 2.47 (t, 2H), 2.68 (s, 3H), 3.32-3.44 (m, 2H), 4.90 (d, 1H), 5.50 (bs, 1H), 5.76 (d, 1H), 6.96-7.34 (m, 9H), 7.68 (bs, 3H).

| **Ex.** | **Compound** | **¹H NMR** | **m/z** | **SM** |
|---|---|---|---|---|
| **E1** | N-(3-Amino-propyl)-N-[1-(6-benzyl-3-methyl-7-oxo-6,7-dihydro- | (300 MHz, DMSO-d₆, 96 °C) δ 0.65 (t, 3H), 1.36-1.50 (m, 1H), 1.60-1.72 (m, 1H), 1.88-1.99 (m, 1H), 2.14-2.26 (m, | m/z 490 (MH⁺) | Method 39 |
| | isothiazolo[4,5-d]pyrimidin-5-yl)-propyl]-4-methyl-benzamide | 1H), 2.35 (s, 3H), 2.47 (t, 2H), 2.68 (s, 3H), 3.32-3.44 (m, 2H), 4.90 (d, 1H), 5.50 (bs, 1H), 5.76 (d, 1H), 6.96-7.34 (m, 9H), 7.68 (bs, 3H). | | |

Compounds of formula (I) have been shown to inhibit the microtubule motor protein HsEg5 in vitro. Inhibitors of Eg5 have been shown to inhibit the formation of a mitotic spindle and therefore for cell division.. Inhibitors of Eg5 have been shown to block cells in the metaphase of mitosis leading to apoptosis of effected cells, and to therefore have anti-proliferative effects. It is believed that Eg5 inhibitors act as modulators of cell division and are expected to be active against neoplastic disease such as carcinomas of the brain, breast, ovary, lung, colon, prostate or other tissues, as well as multiple myeloma leukemias, for example myeloid leukemia, acute lymphoblastic leukemia, chronic myeloid leukemia, chronic lymphocytic leukemia, and lymphomas for example Hodgkins disease and non-Hodgkins lymphoma, tumors of the central and peripheral nervous system, and other tumor types such as melanoma, fibrosarcoma, Ewing's sarcoma and osteosarcoma. Therefore it is believed that the compounds of formula (I) may be used for the treatment of neoplastic disease. Hence the compounds of formula (I) and their salts and their *in vivo* hydrolysable esters are expected to be active against carcinomas of the brain, breast, ovary, lung, colon, prostate or other tissues, as well as leukemias and lymphomas, tumors of the central and peripheral nervous system, and other tumor types such as melanoma, fibrosarcoma and osteosarcoma. The compounds of formula (I) and their salts and their *in vivo* hydrolysable esters are expected to be active against neoplastic disease such as carcinomas of the brain, breast, ovary, lung, colon, prostate or other tissues, as well as multiple myeloma leukemias, for example myeloid leukemia, acute lymphoblastic leukemia, chronic myeloid leukemia, chronic lymphocytic leukemia, and lymphomas for example Hodgkins disease and non-Hodgkins lymphoma, tumors of the central and peripheral nervous system, and other tumor types such as melanoma, fibrosarcoma, Ewing's sarcoma and osteosarcoma. It is expected that the compounds of formula (I) would most likely be used in combination with a broad range of agents but could also be used as a single agent.

Generally, the compounds of formula (I) have been identified in the Malachite Green Assay described herein as having an IC₅₀ value of 100 micromolar or less. For example compound of E1 has an IC₅₀ value of 90 nM.

Compounds provided by this invention should also be useful as standards and reagents in determining the ability of a potential pharmaceutical to inhibit Eg5. These would be provided in commercial kits comprising a compound of this invention.

### Malachite Green Assay

Enzymatic activity of the Eg5 motor and effects of inhibitors was measured using a malachite green assay, which measures phosphate liberated from ATP, and has been used previously to measure the activity of kinesin motors (Hackney and Jiang, 2001). Enzyme was recombinant HsEg5 motor domain (amino acids 1-369-8His) and was added at a final concentration of 6 nM to 100 µl reactions. Buffer consisted of 25 mM PIPES/KOH, pH 6.8, 2 mM MgCl₂, 1 mM EGTA, 1 mM dtt, 0.01% Triton X-100 and 5 µM paclitaxel. Malachite green/ammonium molybdate reagent was prepared as follows: for 800 ml final volume, 0.27 g of Malachite Green (J.T. Baker) was dissolved in 600 ml of H₂O in a polypropylene bottle. 8.4 g ammonium molybdate (Sigma) was dissolved in 200 ml 4N HCl. The solutions were mixed for 20 min and filtered through 0.02 µm filter directly into a polypropylene container. 5 µl of compound diluted in 12% DMSO was added to the wells of 96 well plates. 80 µl of enzyme diluted in buffer solution above was added per well and incubated with compound for 20 min. After this pre-incubation, substrate solution containing 2 mM ATP (final concentration: 300 µM) and 6.053 µM polymerized tubulin (final concentration: 908 nM) in 15 µl of buffer were then added to each well to start reaction. Reaction was mixed and incubated for an additional 20 min at room temperature. The reactions were then quenched by the addition of 150 µl malachite green/ammonium molybdate reagent, and absorbance read at 650 nanometers exactly 5 min after quench using a Spectramax Plus plate reader (Molecular Devices). Data was graphed and IC₅₀s calculated using ExCel Fit (Microsoft).

## Claims

1. A compound, or a pharmaceutically acceptable salt thereof, selected from:
N-(3-amino-propyl)-N-{1-[5-(3-fluoro-benzyl)-3-methyl-4-oxo-4,5-dihydro-isothiazolo[5,4-d]pyrimidin-6-yl]-propyl }-4-methyl-benzamide;
N-(3-amino-propyl)-N-{1-[5-(3-fluoro-benzyl)-3-methyl-4-oxo-4,5-dihydro-isothiazolo[5,4-d]pyrimidin-6-yl]-2-methyl-propyl}-4-methyl-benzamide;
N-(2-amino-ethyl)-N-[1-(5-benzyl-3-methyl-4-oxo-4,5-dihydro-isothiazolo[5,4-d]pyrimidin-6-yl)-2-methyl-propyl]-4-bromo-benzamide;
N-(2-amino-ethyl)-N-[1-(5-benzyl-3-methyl-4-oxo-4,5-dihydro-isothiazolo[5,4-d]pyrimidin-6-yl)-2-methyl-propyl]-4-methyl-benzamide;
N-(2-amino-ethyl)-N-[1-(5-benzyl-3-methyl-4-oxo-4,5-dihydro-isothiazolo[5,4-d]pyrimidin-6-yl)-2-methyl-propyl]-3-fluoro-4-methyl-benzamide;
N-(3-amino-propyl)-N-[1-(5-benzyl-3-methyl-4-oxo-4,5-dihydro-isothiazolo[5,4-d]pyrimidin-6-yl)-2-methyl-propyl]-3-fluoro-4-methyl-benzamide;
N-(3-amino-propyl)-N-[1-(5-benzyl-3-methyl-4-oxo-4,5-dihydro-isothiazolo[5,4-d]pyrimidin-6-yl)-2-methyl-propyl]-4-bromo-benzamide;
N-[1-(5-benzyl-3-methyl-4-oxo-4,5-dihydro-isothiazolo[5,4-d]pyrimidin-6-yl)-2-methyl-propyl]-N-(3-dimethylamino-propyl)-4-methyl-benzamide;
N-[1-(5-benzyl-3-methyl-4-oxo-4,5-dihydro-isothiazolo[5,4-d]pyrimidin-6-yl)-2-methyl-propyl]-N-(3-dimethylamino-propyl)-4-bromo-benzamide;
N-[1-(5-benzyl-3-methyl-4-oxo-4,5-dihydro-isothiazolo[5,4-d]pyrimidin-6-yl)-2-methyl-propyl]-N-(3-dimethylamino-propyl)-3-fluoro-4-methyl-benzamide;
N-(3-amino-propyl)-N-{1-[5-(3-fluoro-benzyl)-3-methyl-4-oxo-4,5-dihydro-isoxazolo [5,4-d]pyrimidin-6-yl]-2-methyl-propyl}-4-methyl-benzamide; and
N-(3-amino-propyl)-N-[1-(6-benzyl-3-methyl-7-oxo-6,7-dihydro-isothiazolo[4,5-d]pyrimidin-5-yl)-propyl]-4-methyl-benzamide.

2. A compound, or a pharmaceutically acceptable salt thereof, as claimed in claim 1 which is N-(3-amino-propyl)-N- {1-[5-(3-fluoro-benzyl)-3-methyl-4-oxo-4,5-dihydro-isothiazolo[5,4-d]pyrimidin-6-yl]-propyl}-4-methyl-benzamide.

3. A compound, or a pharmaceutically acceptable salt thereof, as claimed in claim 1 which is N-(3-amino-propyl)-N-{1-[5-(3-fluoro-benzyl)-3-methyl-4-oxo-4,5-dihydro-isothiazolo[5,4-d]pyrimidin-6-yl]-2-methyl-propyl}-4-methyl-benzamide.

4. A compound, or a pharmaceutically acceptable salt thereof, as claimed in claim 1 which is N-(2-amino-ethyl)-N-[1-(5-benzyl-3-methyl-4-oxo-4,5-dihydro-isothilazolo[5,4-d]pyrimidin-6-yl)-2-methyl-propyl]-4-bromo-benzamide,

5. A compound, or a pharmaceutically acceptable salt thereof, as claimed in claim 1 which is N-(2-amino-ethyl)-N-[1-(5-benzyl-3-methyl-4-oxo-4,5-dihydro-isothiazolo[5,4-d]pyrimidin-6-yl)-2-methyl-propyl]-4-methyl-benzamide.

6. A compound, or a pharmaceutically acceptable salt thereof, as claimed in claim 1 which is N-(2-amino-ethyl)-N-[1-(5-benzyl-3-methyl-4-oxo-4,5-dihydro-isothiazolo[5,4-d]pyrimidin-6-yl)-2-methyl-propyl]-3-fluoro-4-methyl-benzamide.

7. A compound, or a pharmaceutically acceptable salt thereof, as claimed in claim 1 which is N-(3-amino-propyl)-N-[1-(5-benzyl-3-methyl-4-oxo-4,5-dihydro-isothiazolo[5,4-d]pyrimidin-6-yl)-2-methyl-propyl]-3-fluoro-4-methyl-benzamide.

8. A compound, or a pharmaceutically acceptable salt thereof, as claimed in claim 1 which is N-(3-amino-propyl)-N-[1-(5-benzyl-3-methyl-4-oxo-4,5-dihydro-isothiazolo[5,4-d]pyrimidin-6-yl)-2-methyl-propyl]-4-bromo-benzamide.

9. A compound, or a pharmaceutically acceptable salt thereof, as claimed in claim 1 which is N-[1-(5-benzyl-3-methyl-4-oxo-4,5-dihydro-isothiazolo[5,4-d]pyrimidin-6-yl)-2-methyl-propyl]-N-(3-dimethylamino-propyl)-4-methyl-benzamide,

10. A compound, or a pharmaceutically acceptable salt thereof, as claimed in claim 1 which is N-[1-(5-benzyl-3-methyl-4-oxo-4,5-dihydro-isothiazolo[5,4-d]pyimidin-6-yl)-2-methyl-propyl]-N-(3-dimethylamino-propyl)- 4-bromo-benzamide.

11. A compound, or a pharmaceutically acceptable salt thereof, as claimed in claim 1 which is N-[1-(5-benzyl-3-methyl-4-oxo-4,5-dihydro-isothiazolo[5,4-d]pyrimidin-6-yl)-2-methyl-propyl]-N-(3-dimethylamino-propyl)-3-fluoro-4-methyl-benzamide.

12. A compound, or a pharmaceutically acceptable salt thereof, as claimed in claim 1 which is N-(3-amino-propyl)-N-{1-[5-(3-fluoro-benzyl)-3-methyl-4-oxo-4,5-dihydro-isoxazolo [5,4-d]pyrimidin-6-yl]-2-methyl-propyl}-4-methyl-benzamide.

13. A compound, or a pharmaceutically acceptable salt thereof, as claimed in claim 1 which is N-(3-amino-propyl)-N-[1-(6-benzyl-3-methyl-7-oxo-6,7-dihydro-isothiazolo[4,5-d]pyrimidin-5-yl)-propyl]-4-methyl-benzamide.

14. A compound as recited in any one of claims 1 to 13, or a pharmaceutically acceptable salt thereof, for use as a medicament.

15. The use of a compound as recited in any one of claims 1 to 13, or a pharmaceutically acceptable salt thereof, in the manufacture of a medicament for the treatment or prophylaxis of disorders associated with cancer.

16. The use of a compound as recited in any one of claims 1-13, or a pharmaceutically acceptable salt thereof, in the manufacture of a medicament for use in the production of an Eg5 inhibitory effect in a warm-blooded animal such as man.

17. The use of a compound as recited in any one of claims 1-13, or a pharmaceutically acceptable salt thereof, in the manufacture of a medicament for use in the production of an anti-proliferative effect in a warm-blooded animal such as man.

18. The use of a compound as defined in any one of claims 1 to 13, or a pharmaceutically acceptable salt thereof, in the manufacture of a medicament for use in the treatment of carcinomas of the brain, breast, ovary, lung, colon and prostate, multiple myeloma, leukemias, lymphomas, tumors of the central and peripheral nervous system, melanoma, fibrosarcoma, Ewing's sarcoma and osteosarcoma.

19. A pharmaceutical composition comprising a compound as defined in any one of claims 1 to 13, or a pharmaceutically acceptable salt thereof, together with at least one pharmaceutically acceptable carrier, diluent or excipient.

## Patentansprüche

1. Verbindung oder pharmazeutisch annehmbares Salz davon, ausgewählt aus:
N-(3-Aminopropyl)-N-{1-[5-(3-fluorbenzyl)-3-methyl-4-oxo-4,5-dihydroisothiazolo[5,4-d]pyrimidin-6-yl]propyl}-4-methylbenzamid;
N-(3-Aminopropyl)-N-{1-[5-(3-fluorbenzyl)-3-methyl-4-oxo-4,5-dihydroisothiazolo[5,4-d]pyrimidin-6-yl]-2-methylpropyl}-4-methylbenzamid;
N-(2-Aminoethyl)-N-[1-(5-benzyl-3-methyl-4-oxo-4,5-dihydroisothiazolo[5,4-d]pyrimidin-6-yl)-2-methylpropyl]-4-brombenzamid;
N-(2-Aminoethyl)-N-[1-(5-benzyl-3-methyl-4-oxo-4,5-dihydroisothiazolo[5,4-d]pyrimidin-6-yl)-2-methylpropyl]-4-methylbenzamid;
N-(2-Aminoethyl)-N-[1-(5-benzyl-3-methyl-4-oxo-4,5-dihydroisothiazolo[5,4-d]pyrimidin-6-yl)-2-methylpropyl]-3-fluor-4-methylbenzamid;
N-(3-Aminopropyl)-N-[1-(5-benzyl-3-methyl-4-oxo-4,5-dihydroisothiazolo[5,4-d]pyrimidin-6-yl)-2-methylpropyl]-3-fluor-4-methylbenzamid;
N-(3-Aminopropyl)-N-[1-(5-benzyl-3-methyl-4-oxo-4,5-dihydroisothiazolo[5,4-d]pyrimidin-6-yl)-2-methylpropyl]-4-brombenzamid;
N-[1-(5-Benzyl-3-methyl-4-oxo-4,5-dihydroisothiazolo[5,4-d]pyrimidin-6-yl)-2-methylpropyl]-N-(3-dimethylaminopropyl)-4-methylbenzamid;
N-[1-(5-Benzyl-3-methyl-4-oxo-4,5-dihydroisothiazolo[5,4-d]pyrimidin-6-yl)-2-methylpropyl]-N-(3-dimethylaminopropyl)-4-brombenzamid;
N-[1-(5-Benzyl-3-methyl-4-oxo-4,5-dihydroisothiazolo[5,4-d]pyrimidin-6-yl)-2-methylpropyl]-N-(3-dimethylaminopropyl)-3-fluor-4-methylbenzamid;
N-(3-Aminopropyl)-N-{1-[5-(3-fluorbenzyl)-3-methyl-4-oxo-4,5-dihydroisoxazolo[5,4-d]pyrimidin-6-yl]-2-methylpropyl}-4-methylbenzamid; und
N-(3-Aminopropyl)-N-[1-(6-benzyl-3-methyl-7-oxo-6,7-dihydroisothiazolo[4,5-d]pyrimidin-5-yl)-propyl]-4-methylbenzamid.

2. Verbindung oder pharmazeutisch annehmbares Salz davon nach Anspruch 1, bei der es sich um N-(3-Aminopropyl)-N-{1-[5-(3-fluorbenzyl)-3-methyl-4-oxo-4,5-dihydroisothiazolo[5,4-d]pyrimidin-6-yl]propyl}-4-methylbenzamid handelt.

3. Verbindung oder pharmazeutisch annehmbares Salz davon nach Anspruch 1, bei der es sich um N-(3-Aminopropyl)-N-{1-[5-(3-fluorbenzyl)-3-methyl-4-oxo-4,5-dihydroisothiazolo[5,4-d]pyrimidin-6-yl]-2-methylpropyl}-4-methylbenzamid handelt.

4. Verbindung oder pharmazeutisch annehmbares Salz davon nach Anspruch 1, bei der es sich um N-(2-Aminoethyl)-N-[1-(5-benzyl-3-methyl-4-oxo-4,5-dihydroisothiazolo[5,4-d]pyrimidin-6-yl)-2-methylpropyl]-4-brombenzamid handelt.

5. Verbindung oder pharmazeutisch annehmbares Salz davon nach Anspruch 1, bei der es sich um N-(2-Aminoethyl)-N-[1-(5-benzyl-3-methyl-4-oxo-4,5-dihydroisothiazolo[5,4-d]pyrimidin-6-yl)-2-methylpropyl]-4-methylbenzamid handelt.

6. Verbindung oder pharmazeutisch annehmbares Salz davon nach Anspruch 1, bei der es sich um N-(2-Aminoethyl)-N-[1-(5-benzyl-3-methyl-4-oxo-4,5-dihydroisothiazolo[5,4-d]pyrimidin-6-yl)-2-methylpropyl]-3-fluor-4-methylbenzamid handelt.

7. Verbindung oder pharmazeutisch annehmbares Salz davon nach Anspruch 1, bei der es sich um N-(3-Aminopropyl)-N-[1-(5-benzyl-3-methyl-4-oxo-4,5-dihydroisothiazolo[5,4-d]pyrimidin-6-yl)-2-methylpropyl]-3-fluor-4-methylbenzamid handelt.

8. Verbindung oder pharmazeutisch annehmbares Salz davon nach Anspruch 1, bei der es sich um N-(3-Aminopropyl)-N-[1-(5-benzyl-3-methyl-4-oxo-4,5-dihydroisothiazolo[5,4-d]pyrimidin-6-yl)-2-methylpropyl]-4-brombenzamid handelt.

9. Verbindung oder pharmazeutisch annehmbares Salz davon nach Anspruch 1, bei der es sich um N-[1-(5-Benzyl-3-methyl-4-oxo-4,5-dihydroisothiazolo[5,4-d]pyrimidin-6-yl)-2-methylpropyl]-N-(3-dimethylaminopropyl)-4-methylbenzamid handelt.

10. Verbindung oder pharmazeutisch annehmbares Salz davon nach Anspruch 1, bei der es sich um N-[1-(5-Benzyl-3-methyl-4-oxo-4,5-dihydroisothiazolo[5,4-d]pyrimidin-6-yl)-2-methylpropyl]-N-(3-dimethylaminopropyl)-4-brombenzamid handelt.

11. Verbindung oder pharmazeutisch annehmbares Salz davon nach Anspruch 1, bei der es sich um N-[1-(5-Benzyl-3-methyl-4-oxo-4,5-dihydroisothiazolo[5,4-d]pyrimidin-6-yl)-2-methylpropyl]-N-(3-dimethylaminopropyl)-3-fluor-4-methylbenzamid handelt.

12. Verbindung oder pharmazeutisch annehmbares Salz davon nach Anspruch 1, bei der es sich um N-(3-Aminopropyl)-N-{1-[5-(3-fluorbenzyl)-3-methyl-4-oxo-4,5-dihydroisoxazolo[5,4-d]pyrimidin-6-yl]-2-methylpropyl}-4-methylbenzamid handelt.

13. Verbindung oder pharmazeutisch annehmbares Salz davon nach Anspruch 1, bei der es sich um N-(3-Aminopropyl)-N-[1-(6-benzyl-3-methyl-7-oxo-6,7-dihydroisothiazolo[4,5-d]pyrimidin-5-yl)-propyl]-4-methylbenzamid handelt.

14. Verbindung nach einem der Ansprüche 1 bis 13 oder ein pharmazeutisch annehmbares Salz davon zur Verwendung als Medikament.

15. Verwendung einer Verbindung nach einem der Ansprüche 1 bis 13 oder eines pharmazeutisch annehmbaren Salzes davon bei der Herstellung eines Medikaments zur Behandlung oder Prophylaxe von mit Krebs assoziierten Erkrankungen.

16. Verwendung einer Verbindung nach einem der Ansprüche 1 bis 13 oder eines pharmazeutisch annehmbaren Salzes davon bei der Herstellung eines Medikaments zur Verwendung beim Hervorrufen einer Eg5-hemmenden Wirkung in einem Warmblüter wie dem Menschen.

17. Verwendung einer Verbindung nach einem der Ansprüche 1 bis 13 oder eines pharmazeutisch annehmbaren Salzes davon bei der Herstellung eines Medikaments zur Verwendung beim Hervorrufen einer antiproliferativen Wirkung in einem Warmblüter wie dem Menschen.

18. Verwendung einer wie in einem der Ansprüche 1 bis 13 definierten Verbindung oder eines pharmazeutisch annehmbaren Salzes davon bei der Herstellung eines Medikaments zur Verwendung bei der Behandlung von Karzinomen des Hirns, der Brust, der Eierstöcke, der Lunge, des kolons und der Prostata, von multiplen Myelomen, Leukämien, Lymphomen, Tumoren des zentralen und peripheren Nervensystems, Melanomen, Fibrosarkom, Ewing-Sarkom und Osteosarkom.

19. Pharmazeutische Zusammensetzung, enthaltend eine wie in einem der Ansprüche 1 bis 13 definierte Verbindung oder ein pharmazeutisch annehmbares Salz davon zusammen mit wenigstens einem pharmazeutisch annehmbaren Träger, Verdünnungsmittel oder Exzipienten.

## Revendications

1. Composé, ou un sel pharmaceutiquement acceptable de celui-ci, choisi parme :
le N-(3-amino-propyl)-N-{1-[5-(3-fluoro-benzyl)-3-méthyl-4-oxo-4,5-dihydro-isothiazolo[5,4-d]pyrimidin-6-yl]-propyl}-4-méthyl-benzamide ;
le N-(3-amino-propyl)-N-{1-[5-(3-fluoro-benzyl)-3-méthyl-4-oxo-4,5-dihydro-isothiazolo[5,4-d]pyrimidin-6-yl]-2-méthyl-propyl}-4-méthyl-benzamide ;
le N-(2-amino-éthyl)-N-[1-(5-benzyl-3-méthyl-4-oxo-4,5-dihydro-isothiazolo[5,4-d]pyrimidin-6-yl)-2-méthyl-propyl]-4-bromo-benzamide ;
le N-(2-amino-éthyl)-N-[1-(5-benzyl-3-méthyl-4-oxo-4,5-dihydro-isothiazolo[5,4-d]pyrimidin-6-yl)-2-méthyl-propyl]-4-méthyl-benzamide ;
le N-(2-amino-éthyl)-N-[1-(5-benzyl-3-méthyl-4-oxo-4,5-dihydro-isothiazolo[5,4-d]pyrimidin-6-yl)-2-méthyl-propyl]-3-fluoro-4-méthyl-benzamide ;
le N-(3-amino-propyl)-N-[1-(5-benzyl-3-méthyl-4-oxo-4,5-dihydro-isothiazolo[5,4-d]pyrimidin-6-yl)-2-méthyl-propyl]-3-fluoro-4-méthyl-benzamide ;
le N-(3-amino-propyl)-N-[1-(5-benzyl-3-méthyl-4-oxo-4,5-dihydro-isothiazolo[5,4-d]pyrimidin-6-yl)-2-méthyl-propyl]-4-bromo-benzamide ;
le N-[1-(5-benzyl-3-méthyl-4-oxo-4,5-dihydro-isothiazolo[5,4-d]pyrimidin-6-yl)-2-méthylpropyl]-N-(3-diméthylamino-propyl)4-méthyl-benzamide ;
le N-[1-(5-benzyl-3-méthyl-4-oxo-4,5-dihydroisothiazolo[5,4-d]pyrimidin-6-yl)-2-méthylpropyl]-N-(3-diméthylamino-propyl)4-bromo-benzamide ;
le N-[1-(5-benzyl-3-méthyl-4-oxo-4,5-dihydroisothiazolo[5,4-d]pyrimidin-6-yl)-2-méthylpropyl]-N-(3-diméthylamino-propyl)3-fluoro-4-méthyl-benzamide ;
le N-(3-amino-propyl)-N-{1-[5-(3-fluoro-benzyl)-3-méthyl-4-oxo-4,5-dihydro-isoxazolo[5,4-d]pyrimidin-6-yl]-2-méthyl-propyl}-4-méthylbenzamide ; et
le N-(3-amino-propyl)-N-[1-(6-benzyl-3-méthyl-7-oxo-6,7-dihydro-isothiazolo[4,5-d]pyrimidin-5-yl)-propyl]-4-méthyl-benzamide.

2. Composé, ou un sel pharmaceutiquement acceptable de celui-ci, selon la revendication 1, **caractérisé en ce qu'**il s'agit de N-(3-amino-propyl)-N-{1-[5-(3-fluoro-benzyl)-3-méthyl-4-oxo-4,5-dihydroisothiazolo[5,4-d]pyrimidin-6-yl]-propyl}-4-méthyl-benzamide.

3. Composé, ou un sel pharmaceutiquement acceptable de celui-ci, selon la revendication 1, **caractérisé en ce qu'**il s'agit de N-(3-amino-propyl)-N-{1-[5-(3-fluoro-benzyl)-3-méthyl-4-oxo-4,5-dihydroisothiazolo[5,4-d]pyrimidin-6-yl]-2-méthylpropyl}-4-méthyl-benzamide.

4. Composé, ou un sel pharmaceutiquement acceptable de celui-ci, selon la revendication 1, **caractérisé en ce qu'**il s'agit de N-(2-amino-éthyl)-N-[1-(5-benzyl-3-méthyl-4-oxo-4,5-dihydro-isothiazolo[5,4-d]pyrimidin-6-yl)-2-méthyl-propyl]-4-bromo-benzamide.

5. Composé, ou un sel pharmaceutiquement acceptable de celui-ci, selon la revendication 1, **caractérisé en ce qu'**il s'agit de N-(2-amino-éthyl)-N-[1-(5-benzyl-3-méthyl-4-oxo-4,5-dihydro-isothiazolo[5,4-d]pyrimidin-6-yl)-2-méthyl-propyl]-4-méthylbenzamide.

6. Composé, ou un sel pharmaceutiquement acceptable de celui-ci, selon la revendication 1, **caractérisé en ce qu'**il s'agit de N-(2-amino-éthyl)-N-[1-(5-benzyl-3-méthyl-4-oxo-4,5-dihydro-isothiazolo[5,4-d]pyrimidin-6-yl)-2-méthyl-propyl]-3-fluoro-4-méthyl-benzamide.

7. Composé, ou un sel pharmaceutiquement acceptable de celui-ci, selon la revendication 1, **caractérisé en ce qu'**il s'agit de N-(3-amino-propyl)-N-[1-(5-benzyl-3-méthyl-4-oxo-4,5-dihydro-isothiazolo[5,4-d]pyrimidin-6-yl)-2-méthyl-propyl]-3-fluoro-4-méthyl-benzamide.

8. Composé, ou un sel pharmaceutiquement acceptable de celui-ci, selon la revendication 1, **caractérisé en ce qu'**il s'agit de N-(3-amino-propyl)-N-[1-(5-benzyl-3-méthyl-4-oxo-4,5-dihydro-isothiazolo[5,4-d]pyrimidin-6-yl)-2-méthyl-propyl]-4-bromo-benzamide.

9. Composé, ou un sel pharmaceutiquement acceptable de celui-ci, selon la revendication 1, **caractérisé en ce qu'**il s'agit de N-[1-(5-benzyl-3-méthyl-4-oxo-4,5-dihydro-isothiazolo[5,4-d]pyrimidin-6-yl)-2-méthyl-propyl]-N-(3-diméthylamino-propyl)-4-méthyl-benzamide.

10. Composé, ou un sel pharmaceutiquement acceptable de celui-ci, selon la revendication 1, **caractérisé en ce qu'**il s'agit de N-[1-(5-benzyl-3-méthyl-4-oxo-4,5-dihydro-isothiazolo[5,4-d]pyrimidin-6-yl)-2-méthyl-propyl]-N-(3-diméthylamino-propyl)-4-bromo-benzamide.

11. Composé, ou un sel pharmaceutiquement acceptable de celui-ci, selon la revendication 1, **caractérisé en ce qu'**il s'agit de N-[1-(5-benzyl-3-méthyl-4-oxo-4,5-dihydro-isothiazolo[5,4-d]pyrimidin-6-yl)-2-méthyl-propyl]-N-(3-diméthylamino-propyl)-3-fluoro-4-méthyl-benzamide.

12. Composé, ou un sel pharmaceutiquement acceptable de celui-ci, selon la revendication 1, **caractérisé en ce qu'**il s'agit de N-(3-amino-propyl)-N-{1-[5-(3-fluoro-benzyl)-3-méthyl-4-oxo-4,5-dihydro-isoxazolo[5,4-d]pyrimidin-6-yl]-2-méthyl-propyl}-4-méthyl-benzamide.

13. Composé, ou un sel pharmaceutiquement acceptable de celui-ci, selon la revendication 1, **caractérisé en ce qu'**il s'agit de N-(3-amino-propyl)-N-[1-(6-benzyl-3-méthyl-7-oxo-6,7-dihydro-isothiazolo[4,5-d]pyrimidin-5-yl)-propyl]-4-méthyl-benzamide.

14. Composé selon l'une quelconque des revendications 1 à 13, ou un sel pharmaceutiquement acceptable de celui-ci, destiné à être utilisé comme médicament.

15. Utilisation d'un composé selon l'une quelconque des revendications 1 à 13, ou d'un sel pharmaceutiquement acceptable de celui-ci, dans la fabrication d'un médicament destiné au traitement ou à la prophylaxie de troubles associés au cancer.

16. Utilisation d'un composé selon l'une quelconque des revendications 1 à 13, ou d'un sel pharmaceutiquement acceptable de celui-ci, dans la fabrication d'un médicament destiné à être utilisé dans la production d'un effet inhibiteur d'Eg5 chez un animal à sang chaud tel que l'homme.

17. Utilisation d'un composé selon l'une quelconque des revendications 1 à 13, ou d'un sel pharmaceutiquement acceptable de celui-ci, dans la fabrication d'un médicament destiné à être utilisé dans la production d'un effet antiprolifératif chez un animal à sang chaud tel que l'homme.

18. Utilisation d'un composé tel que défini dans l'une quelconque des revendications 1 à 13, ou d'un sel pharmaceutiquement acceptable de celui-ci, dans la fabrication d'un médicament destiné à être utilisé dans le traitement des carcinomes du cerveau, du sein, de l'ovaire, du poumon, du côlon et de la prostate, du myélome multiple, des leucémies, des lymphomes, des tumeurs du système nerveux central et périphérique, du mélanome, du fibrosarcome, du sarcome d'Ewing et de l'ostéosarcome.

19. Composition pharmaceutique comprenant un composé tel que défini dans l'une quelconque des revendications 1 à 13, ou un sel pharmaceutiquement acceptable de celui-ci, conjointement avec au moins un support, un diluant ou un excipient pharmaceutiquement acceptable.
